# EUROPEAN PATENT SPECIFICATION

(11) **EP 3 581 180 B1**
(45) Date of publication and mention of the grant of the patent: **04.08.2021**
(21) Application number: 19165084.5
(22) Date of filing: 19.06.2015
(51) Int. Cl.: A61K 31/4709, A61K 9/00, A61K 9/20, A61K 9/46, A61P 31/04, A61K 31/133, A61P 31/00

(54) **ANTIMICROBIAL COMPOSITIONS WITH EFFERVESCENT AGENTS**
ANTIMIKROBIELLE ZUSAMMENSETZUNGEN MIT BRAUSEMITTEL
COMPOSITIONS ANTIMICROBIENNES AYANT DES AGENTS EFFERVESCENTS

(30) Priority: 20.06.2014 US 201462014786 P
(43) Date of publication of application: 18.12.2019
(62) Divisional of application: 15810507.2
(73) Proprietor: Melinta Subsidiary Corp., Morristown, NJ 07960 (US)
(72) Inventor: LI, Danping, Middlebury, CT 06762 (US)
(74) Representative: Bassil, Nicholas Charles

(56) References cited:
- WO-A2-2010/096551
- US-A1- 2012 065 186
- Robert E. Lee: "Effervescent tablets", Amerilab Technologies, 2012, pages 1-4, XP055440338, Retrieved from the Internet: URL:https://www.amerilabtech.com/wp-conten t/uploads/2012/01/EffervescentTabletsKeyFa cts.pdf [retrieved on 2018-01-12]

## Description

### REFERENCE TO RELATED APPLICATIONS

This application claims the benefit of priority of United States Provisional Patent Application No. 62/014,786, filed June 20, 2014.

### FIELD OF THE INVENTION

The present invention relates to pharmaceutical compositions comprising a quinolone carboxylic acid derivative antimicrobial agent and an effervescent agent. These compositions have improved gastrointestinal tolerability. These compositions are useful for oral administration, for treating, preventing, or reducing the risk of microbial infections.

### BACKGROUND

Antibiotics are widely available, inexpensive, and seen as routine agents for combatting bacterial infections. However, over-prescription and failures in patient compliance (*e.g.*, to complete a full cycle of antibiotics as prescribed) has given rise to antibiotic-resistant bacteria, known as "superbugs." *See, e.g.*, "Antibiotics Crisis Prompts Rethinking On Risks, Rewards," Medscape Mar. 18, 2013. Reports of the steady rise in reports of "superbugs," in combination with the decreasing number of approvals of new systemic antibiotics has prompted international concern. *See id.* For example, methicillin-resistant Staphylococcus aureus ("MRSA"), is estimated to kill about 40,000 people every year in the United States and Europe, while totally drug resistant tuberculosis, untreatable strains of gonorrhea and "super superbugs" having the New Delhi metallo-beta-lactamase (NDM 1) mutation are become more prevalent around the globe. *Id.* Accordingly, a need exists for new antibiotic drugs.

An appropriate pharmaceutical carrier system is generally a requirement for the safe and effective delivery of a pharmaceutical drug active. The entire pharmaceutical composition, *i.e.* the pharmaceutical drug active formulated in a pharmaceutical carrier, can affect the bioavailability and also the pharmacokinetics and pharmacodynamics of the drug active. It is therefore important that a pharmaceutical composition be carefully developed and manufactured to deliver the desired pharmaceutical drug active in a safe and effective manner.

The delivery of antimicrobial agents for treating or preventing microbial infections can present special challenges. To provide therapeutic efficacy, it is generally desired that the antimicrobial agent be administered to the patient to achieve systemic concentrations in the bloodstream or target organs above a minimum inhibitory concentration for a sufficient time against the particular microbial organism or organisms being targeted. Consequently, an antimicrobial agent that otherwise exhibits an effective antimicrobial profile *in vitro* can be ineffective, or even harmful, unless properly formulated for *in vivo* administration.

The challenge of developing suitable antimicrobial compositions is further complicated for the development of compositions for oral administration. For example, antimicrobial agents can affect the flora of the gastrointestinal tract, particularly in the small and large intestine, which can result in untoward gastrointestinal side effects such as diarrhea, flatulence, and general abdominal discomfort. Furthermore, antimicrobial agents can exert a selective pressure on the resident bacteria of the gastrointestinal tract resulting in the emergence of bacteria resistant to the antimicrobial agent. The gastrointestinal side effects can affect patient compliance with the antimicrobial dosing regimen, therefore compromising drug effectiveness and potentially subjecting the patient to recurring or more resistant infections. In severe cases, the gastrointestinal side effects can result in disruption or even total discontinuation of therapy.

The potential development of bacterial resistance is a broader public health concern. In certain instances, because of the selective pressure of the antimicrobial agent on the bacterial flora of the gastrointestinal tract, the patient receiving antimicrobial therapy can become colonized by bacteria resistant to the antimicrobial agent. The resultant resistant bacteria can not only be harmful to the patient but can be further disseminated into and become established in the community.

It is believed by those skilled in the art that the gastrointestinal side effects and resistance problems are caused by residual antimicrobial agent that is not absorbed from the gastrointestinal tract into the blood stream. In such instances, it is believed that the antimicrobial agent that remains in and passes through the gastrointestinal tract, particularly the antimicrobial agent that passes into the cecum and colon, can cause these gastrointestinal side effects and resistance problems. Additionally, the antimicrobial agent that is absorbed into the blood stream can, depending on the particular pharmacokinetics of the antimicrobial agent, be excreted from the liver through the bile and back into the gastrointestinal tract to further augment the gastrointestinal side effects and resistance problems.

WO2010/096551 describes pharmaceutical compositions comprising a quinolone carboxylic acid compound and an absorption enhancer, optionally further comprising a basifier or crystallization inhibitor or both a basifier and a crystallization inhibitor.

There is thus a need to develop formulations which minimize the presence of residual or unwanted antimicrobial agent in the gastrointestinal tract. In particular there is a need to develop formulations which minimize the presence of residual or unwanted antimicrobial agent in the cecum and colon. There is also a need to develop oral formulations which minimize the undesirable gastrointestinal side effects and the development of antibiotic resistance.

Therefore, the present invention addresses the foregoing and other needs by providing compositions as defined herein.

The invention is defined by the claims. Any subject-matter falling outside the scope of the claims is provided for information purposes only.

Any references in the description to methods of treatment refer to the devices, compounds, pharmaceutical compositions and medicaments of the present invention for use in a method for treatment of the human or animal body by therapy.

### BRIEF DESCRIPTION OF THE FIGURES

FIG. 1 shows a powder X-ray diffraction (XRPD) pattern of crystalline D-glucitol, 1-deoxy-1-(methylamino)-, 1-(6-amino-3,5-difluoropyridin-2-yl)-8-chloro-6-fluoro-1,4-dihydro-7-(3 -hydroxyazetidin-1 -yl)-4-oxo-3 -quinolinecarboxylate.
FIG. 2 shows a powder X-ray diffraction pattern of crystalline D-glucitol, 1-deoxy-1-(methylamino)-, 1-(6-amino-3,5-difluoropyridin-2-yl)-8-chloro-6-fluoro-1,4-dihydro-7-(3-hydroxyazetidin-1-yl)-4-oxo-3-quinolinecarboxylate trihydrate.

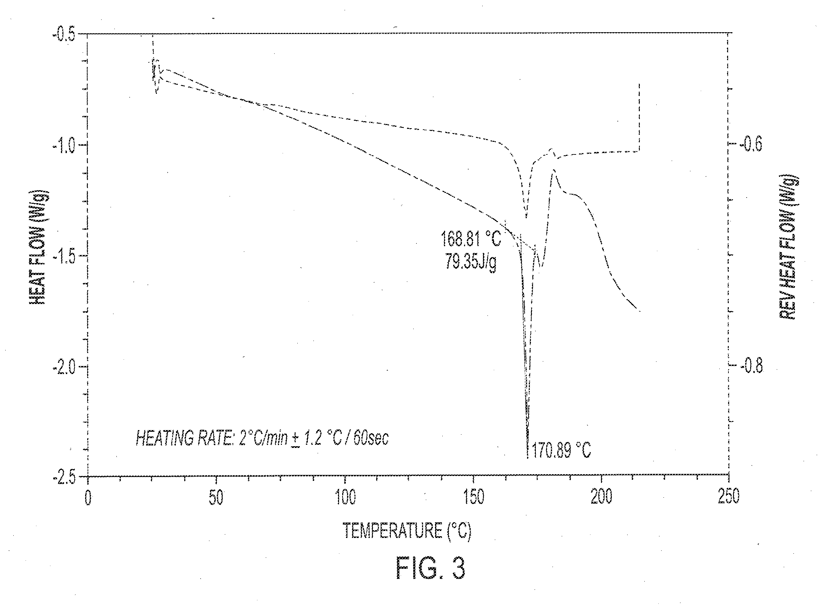

FIG. 3 shows a Modulated Differential Scanning Calorimetry (mDSC) thermogram of crystalline D-glucitol, 1-deoxy-1-(methylamino)-, 1-(6-amino-3,5-difluoropyridin-2-yl)-8-chloro-6-fluoro-1,4-dihydro-7-(3-hydroxyazetidin-1-yl)-4-oxo-3-quinolinecarboyxlate.
FIG. 4 shows a dissolution profile for certain formulations according to the present disclosure.

### SUMMARY OF THE INVENTION

The present invention is based, in part, on the surprising discovery that pharmaceutical compositions comprising a quinolone carboxylic acid derivative antimicrobial agent and an effervescent agent have improved gastrointestinal tolerability. These compositions are useful for oral administration, for treating, preventing, or reducing the risk of microbial infections.

The invention provides a pharmaceutical composition comprising: (a) a quinolone carboxylic acid derivative or a pharmaceutically acceptable salt or ester thereof; and (b) an effervescent agent; wherein said quinolone carboxylic acid derivative corresponds to the following compound (A) 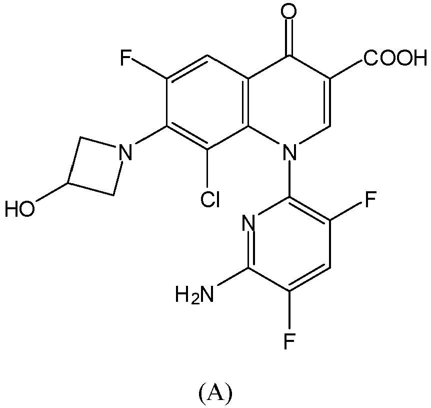 or a pharmaceutically acceptable salt or ester thereof, and wherein said effervescent agent comprises a mixture of sodium bicarbonate, sodium dihydrogen phosphate, and citric acid.

In some embodiments, the quinolone carboxylic acid derivative is a D-glucitol, 1-deoxy-1-(methylamino)-, 1-(6-amino-3,5-difluoro-2-pyridinyl)-8-chloro-6-fluoro-1,4-dihydro-7-(3-hydroxy-1-azetidinyl)-4-oxo-3-quinolinecarboxylate.

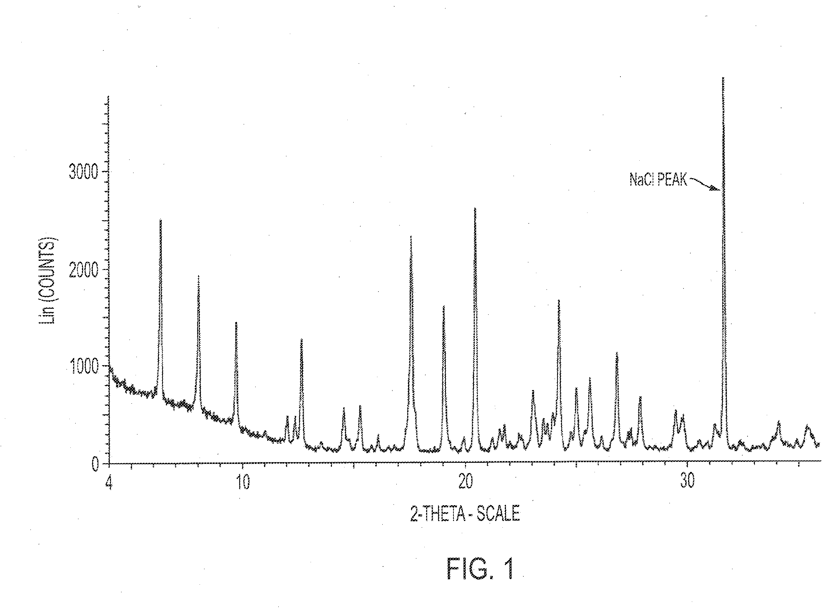

In some embodiments, the quinolone carboxylic acid derivative is a crystalline D-glucitol, 1-deoxy-1-(methylamino)-, 1-(6-amino-3,5-difluoro-2-pyridinyl)-8-chloro-6-fluoro-1,4-dihydro-7-(3-hydroxy-1-azetidinyl)-4-oxo-3-quinolinecarboxylate characterized, wherein the pattern is obtained from a copper radiation source with Cu-Kα radiation, by the x-ray powder diffraction pattern shown in FIGURE 1.

In some embodiments, the quinolone carboxylic acid derivative is D-glucitol, 1-deoxy-1-(methylamino)-, 1-(6-amino-3,5-difluoro-2-pyridinyl)-8-chloro-6-fluoro-1,4-dihydro-7-(3-hydroxy-1-azetidinyl)-4-oxo-3-quinolinecarboxylate trihydrate.

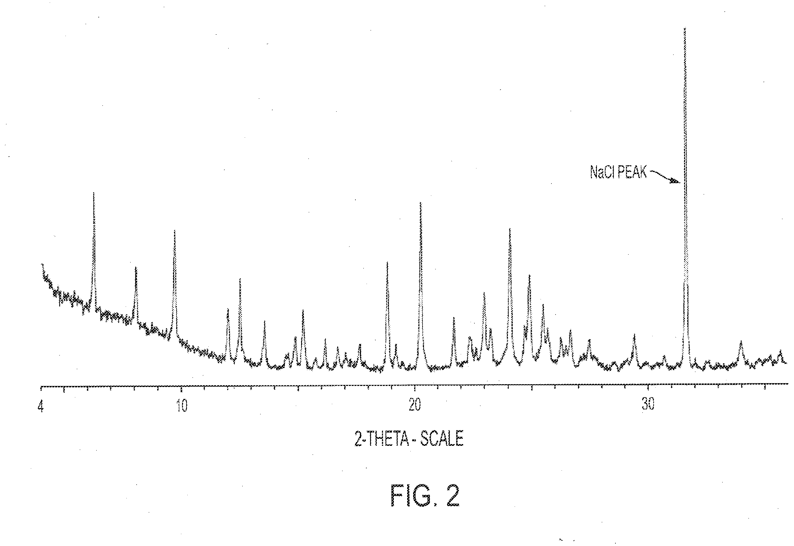

In some embodiments, the quinolone carboxylic acid derivative is crystalline D-glucitol, 1-deoxy-1-(methylamino)-, 1-(6-amino-3,5-difluoro-2-pyridinyl)-8-chloro-6-fluoro-1,4-dihydro-7-(3-hydroxy-1-azetidinyl)-4-oxo-3-quinolinecarboxylate trihydrate, characterized, when measured at about 25 °C with Cu-Kα radiation, by the x-ray powder diffraction pattern shown in FIGURE 2.

The effervescent agent comprises a mixture of sodium bicarbonate, sodium dihydrogen phosphate (also known as sodium phosphate monobasic or monosodium phosphate) and citric acid.

In some embodiments, the pharmaceutical composition further comprises a polyhydroxyamine compound.

In some embodiments, the polyhydroxyamine compound is meglumine.

In another aspect, the invention provides a pharmaceutical composition comprising: (a) from about 0.01% to about 80% by weight of a quinolone carboxylic acid derivative, or a pharmaceutically acceptable salt thereof, as compared to the total weight of the composition on an acid basis; and (b) from about 0.1% to about 50% by weight of an effervescent agent, as compared to the total weight of the composition; wherein the quinolone carboxylic acid derivative, or a pharmaceutically acceptable salt thereof, and the effervescent agent are as described herein.

In another aspect, the invention provides a pharmaceutical composition comprising: (a) from about 0.01% to about 80% by weight of a quinolone carboxylic acid derivative, or a pharmaceutically acceptable salt thereof, as compared to the total weight of the composition on an acid basis; (b) from about 0.1% to about 50% by weight of meglumine, as compared to the total weight of the composition; and (c) from about 0.1% to about 50% by weight of an effervescent agent, as compared to the total weight of the composition; wherein the quinolone carboxylic acid derivative, or a pharmaceutically acceptable salt thereof, and the effervescent agent are as described herein.

In another aspect, the invention provides pharmaceutical composition comprising: (a) from about 0.01% to about 50% by weight of delafloxacin, or a pharmaceutically acceptable salt thereof, as compared to the total weight of the composition on an acid basis; and (b) from about 0.1% to about 50% by weight of an effervescent agent, as compared to the total weight of the composition, the effervescent agent comprising the mixture of sodium bicarbonate, sodium dihydrogen phosphate, and citric acid.

In another aspect, the invention provides a pharmaceutical composition comprising: (a) from about 0.01% to about 50% by weight of delafloxacin, or a pharmaceutically acceptable salt thereof, as compared to the total weight of the composition on an acid basis; (b) from about 0.1% to about 50% by weight of meglumine, as compared to the total weight of the composition; and (c) from about 0.1% to about 50% by weight of an effervescent agent, as compared to the total weight of the composition, the effervescent agent comprising the mixture of sodium bicarbonate, sodium dihydrogen phosphate, and citric acid.

In another aspect, the invention provides pharmaceutical composition comprising: (a) from about 100 mg to about 750 mg of delafloxacin, on an acid active basis; and (b) from about 100 mg to about 500 mg of an effervescent agent comprising the mixture of sodium bicarbonate, sodium dihydrogen phosphate, and citric acid.

In another aspect, the invention provides pharmaceutical composition comprising: (a) about 400 mg of delafloxacin meglumine, on an acid active basis; and (b) about 150 mg of an effervescent agent comprising the mixture of sodium bicarbonate, sodium dihydrogen phosphate, and citric acid.

In another aspect, the invention provides a pharmaceutical composition comprising: (a) about 450 mg of delafloxacin, (b) about 199 mg of meglumine; and (c) about 150 mg of an effervescent agent comprising the mixture of sodium bicarbonate, sodium phosphate, and citric acid.

In some embodiments, the composition has improved gastrointestinal tolerability.

In some embodiments, the composition has reduced potential to cause gastrointestinal side effects.

In some embodiments, the composition has improved gastrointestinal tolerability or has reduced potential to cause gastrointestinal side effects as evaluated in a mini pig model.

In some embodiments, the composition has improved gastrointestinal tolerability or has reduced potential to cause gastrointestinal side effects as evaluated in humans.

In some embodiments, the composition has diminished potential to cause bacterial resistance.

In some embodiments, the composition is in the form of a dry mixture, an aqueous solution, an aqueous suspension, a non-aqueous solution, a non-aqueous suspension, or an emulsion.

In some embodiments, the composition is a unit dosage.

In some embodiments, the composition is in the form of a tablet.

In some embodiments, the composition is in the form of a single layer tablet.

In some embodiments, the composition is in the form of a bilayer tablet comprising a first layer and a second layer. In some embodiments, the first layer comprises the quinolone carboxylic acid derivative or a pharmaceutically acceptable salt thereof and the second layer comprises the effervescent agent. In some embodiments, the second layer is a delayed release layer, where the second layer is released in the gastrointestinal tract downstream from the stomach.

In some embodiments, the composition is in the form of a capsule.

In another aspect, the disclosure provides a method for treating, preventing, or reducing the risk of a bacterial infection comprising administering to a patient in need thereof an effective amount of the composition described herein.

In some embodiments, the disclosure provides a method for treating a bacterial infection comprising administering to a patient in need thereof an effective amount of the composition described herein. In some embodiments, the disclosure provides a method for preventing a bacterial infection comprising administering to a patient in need thereof an effective amount of the composition described herein. In some embodiments, the disclosure provides a method for reducing the risk of a bacterial infection comprising administering to a patient in need thereof an effective amount of the composition described herein.

In some embodiments, the method has improved gastrointestinal tolerability. In some embodiments, the method has reduced potential to cause gastrointestinal side effects.

In one aspect, the disclosure provides a method for treating, preventing, or reducing the risk of a bacterial infection, while reducing the potential for causing gastrointestinal side effects, comprising administering to a patient in need thereof an effective amount of the composition described herein.

In one aspect, the disclosure provides a method for treating, preventing, or reducing the risk of a bacterial infection, while reducing the potential for causing bacterial resistance, comprising administering to a patient in need thereof an effective amount of the composition described herein.

### DETAILED DESCRIPTION OF THE INVENTION

In the present invention as defined in the appended claims, it has surprisingly been found that the combination of a quinolone carboxylic acid derivative antimicrobial agent and an effervescent agent are found to provide a desired balance of antimicrobial activity and improved gastrointestinal toleration and also may reduce the potential for bacterial resistance. Without being limited by theory, the final drug product formulation may be the result of a complex interplay of solubility, stability, and toleration.

The present disclosure relates to pharmaceutical compositions comprising a quinolone carboxylic acid derivative and an effervescent agent. It has been found that these compositions have improved gastrointestinal tolerability. These compositions are useful for oral administration, for treating, preventing, or reducing the risk of a microbial infection.

The present disclosure relates to a pharmaceutical composition comprising a quinolone carboxylic acid derivative or a pharmaceutically acceptable salt or ester thereof, and an effervescent agent. These compositions are useful for oral administration for treating, preventing, or reducing the risk of infection. These compositions have enhanced patient gastrointestinal toleration when administered orally. The present invention provides compositions having enhanced gastrointestinal toleration compared to what would otherwise be achievable not employing the present invention. Enhanced gastrointestinal patient toleration is important, because the invention provides compositions that are safe and well tolerated. It is not sufficient for a pharmaceutical composition to be efficacious, it is important that efficacy be achieved at an appropriate safety and toleration level. Therefore, the compositions of the present invention provide an advantage over the state of the art.

In one aspect, the disclosure relates to a method for treating, preventing, or reducing the risk of a bacterial infection in a patient in need thereof a composition as taught herein, wherein said composition has improved gastrointestinal tolerability.

In one aspect, the disclosure relates to a method for treating, preventing, or reducing the risk of a bacterial infection in a patient in need thereof a composition as taught herein, wherein said composition has the reduced potential to cause gastrointestinal side effects. The gastrointestinal side effects in the foregoing are selected from diarrhea, flatulence, nausea, vomiting, abdominal pain, dyspepsia, belching, bloating, gastritis, and general abdominal discomfort.

In one aspect, the disclosure relates to a method for treating, preventing, or reducing the risk of a bacterial infection in a patient in need thereof a composition as taught herein, wherein said composition has the reduced potential to cause gastrointestinal side effects and wherein said composition has the reduced potential to cause bacterial resistance. The gastrointestinal side effects in the foregoing are selected from diarrhea, flatulence, nausea, vomiting, abdominal pain, dyspepsia, belching, bloating, gastritis, and general abdominal discomfort.

The present invention relates to a composition wherein said quinolone carboxylic acid derivative corresponds to the following compound (A), 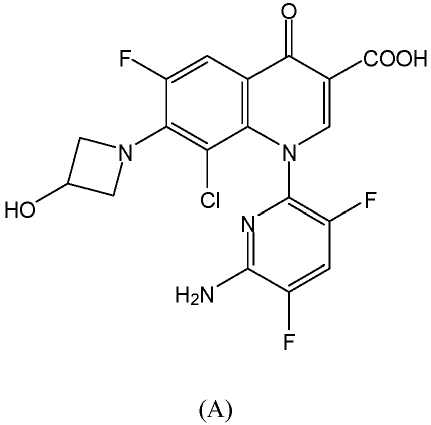 or a pharmaceutically acceptable salt or ester thereof.

In other embodiments, the present invention relates to a composition wherein said quinolone carboxylic acid derivative is a D-glucitol, 1-deoxy-1-(methylamino)-, 1-(6-amino-3,5-difluoro-2-pyridinyl)-8-chloro-6-fluoro-1,4-dihydro-7-(3-hydroxy-1-azetidinyl)-4-oxo-3-quinolinecarboxylate. In other embodiments, the present invention relates to a composition wherein said quinolone carboxylic acid derivative is a crystalline D-glucitol, 1-deoxy-1-(methylamino)-, 1-(6-amino-3,5-difluoro-2-pyridinyl)-8-chloro-6-fluoro-1,4-dihydro-7-(3-hydroxy-1-azetidinyl)-4-oxo-3-quinolinecarboxylate. In yet other embodiments, the present invention relates to a composition wherein said quinolone carboxylic acid derivative is a crystalline D-glucitol, 1-deoxy-1-(methylamino)-, 1-(6-amino-3,5-difluoro-2-pyridinyl)-8-chloro-6-fluoro-1,4-dihydro-7-(3-hydroxy-1-azetidinyl)-4-oxo-3-quinolinecarboxylate characterized, wherein the pattern is obtained from a copper radiation source with Cu-Kα radiation, by the x-ray powder diffraction pattern shown in FIGURE 1.

In other embodiments, the present invention relates to a composition wherein said quinolone carboxylic acid derivative is D-glucitol, 1-deoxy-1-(methylamino)-, 1-(6-amino-3,5-difluoro-2-pyridinyl)-8-chloro-6-fluoro-1,4-dihydro-7-(3-hydroxy-1-azetidinyl)-4-oxo-3-quinolinecarboxylate trihydrate. In other embodiments, the present invention relates to a composition wherein said quinolone carboxylic acid derivative is crystalline D-glucitol, 1-deoxy-1-(methylamino)-, 1-(6-amino-3,5-difluoro-2-pyridinyl)-8-chloro-6-fluoro-1,4-dihydro-7-(3-hydroxy-1-azetidinyl)-4-oxo-3-quinolinecarboxylate trihydrate. In yet other embodiments, the present invention relates to a composition wherein said quinolone carboxylic acid derivative is crystalline D-glucitol, 1-deoxy-1-(methylamino)-, 1-(6-amino-3,5-difluoro-2-pyridinyl)-8-chloro-6-fluoro-1,4-dihydro-7-(3-hydroxy-1-azetidinyl)-4-oxo-3-quinolinecarboxylate trihydrate characterized, when measured at about 25 °C with Cu-Kα radiation, by the x-ray powder diffraction pattern shown in FIGURE 2.

The invention relates to a pharmaceutical composition wherein said effervescent agent is that releases carbon dioxide upon contact with water or a gastrointestinal fluid.

The present invention relates to a composition wherein said composition provides a measurable improvement in gastrointestinal toleration. In other embodiments, the present invention relates to a composition wherein said gastrointestinal toleration is measured in an animal model. In other embodiments, the present invention relates to a composition wherein said gastrointestinal toleration is measured in a mini-pig model. In other embodiments, the present invention relates to a composition wherein said gastrointestinal toleration is measured in humans.

In other embodiments, the present invention relates to a pharmaceutical composition comprising (a) from about 100 mg to about 500 mg of delafloxacin meglumine, and (b) from about 100 mg to about 500 mg of an effervescent agent, wherein the effervescent agent is as described herein.

In other embodiments, the present disclosure relates to a method for treating, preventing, or reducing the risk of a bacterial infection comprising administering to a patient in need thereof a composition as described herein.

### 1. Definitions

The term "patient", as used herein, means the human or animal (in the case of an animal, more typically a mammal) subject. The term "mammal" includes a human, mouse, rat, guinea pig, dog, cat, horse, cow, pig, or non-human primate, such as a monkey, chimpanzee, baboon or rhesus. In one embodiment, the mammal is a human. The patient is usually one that is in need of the compositions or methods described herein. "In need of," can mean that the patient has or is diagnosed as having an infection, *e.g.*, a microbial infection, or that the patient is at risk of contracting an infection due to an injury, a medical or surgical procedure, or microbial exposure, or could be in a position that could subject the patient to such exposure. Such infections can be due to, *e.g.*, a skin infection, nosocomial pneumonia, post-viral pneumonia, an abdominal infection, a urinary tract infection, bacteremia, septicemia, endocarditis, an atrio-ventricular shunt infection, a vascular access infection, meningitis, infection due to surgical or invasive medical procedures, a peritoneal infection, a bone infection, a joint infection, a methicillin-resistant *Staphylococcus aureus* infection, a vancomycin-resistant *Enterococci* infection, a linezolid-resistant organism infection, tuberculosis, a quinolone resistant Gram-positive infection, a ciprofloxacin resistant methicillin resistant (MRSA) infection, bronchitis, a complicated skin and skin structure infection (cSSSI), an uncomplicated skin and skin structure infection (uSSSI), a community respiratory-tract infection, and a multi-drug resistant (MDR) Gram-negative infection.

The term "preventing", as used herein, means, *e.g.,* to completely or almost completely stop an infection from occurring, for example when the patient is predisposed to an infection or at risk of contracting an infection.

The term "reducing the risk of", as used herein means, *e.g.,* to lower the likelihood or probability of an infection occurring, for example when the patient is predisposed to an infection or at risk of contracting an infection.

The term "treating" as used herein means, *e.g.,* to cure, inhibit, arrest the development, relieve the symptoms or effects of, ameliorating, or cause the regression of an infection in a patient having an infection.

It should be recognized that the terms "preventing", "reducing the risk of", and "treating" are not intended to limit the scope of the invention and that there can be overlap amongst these terms.

As used herein, the term "effective amount" means an amount of a pharmaceutically active compound, *i.e.* a drug active, *e.g.,* a quinolone carboxylic acid derivative or pharmaceutically acceptable salt thereof, given to a recipient patient sufficient to elicit biological activity, for example, anti-infective activity, *e.g.,* anti-microbial activity.

The term "prophylactically effective amount" means an amount of a pharmaceutically active compound, *i.e.* a drug active, *e.g.,* a quinolone carboxylic acid derivative given to a recipient patent sufficient to prevent or reduce the risk of a microbial infection.

As used herein, the phrase "pharmaceutically acceptable" refers to those compounds, materials, compositions, carriers, and/or dosage forms which are, within the scope of sound medical judgment, suitable for use in contact with the tissues of human beings and animals without excessive toxicity, irritation, allergic response, or other problem or complication, commensurate with a reasonable benefit/risk ratio.

As used herein, "pharmaceutically acceptable salts" refer to derivatives of the disclosed compounds wherein the parent compound is modified by making acid or base salts thereof. Examples of pharmaceutically acceptable salts include, but are not limited to, mineral or organic acid salts of basic residues such as amines, alkali or organic salts of acidic residues such as carboxylic acids, and the like. The pharmaceutically acceptable salts include the conventional non-toxic salts or the quaternary ammonium salts of the parent compound formed, for example, from non-toxic inorganic or organic acids. For example, such conventional non-toxic salts include, but are not limited to, those derived from inorganic and organic acids selected from 2-acetoxybenzoic, 2-hydroxyethane sulfonic, acetic, ascorbic, benzene sulfonic, benzoic, bicarbonic, carbonic, citric, edetic, ethane disulfonic, ethane sulfonic, fumaric, glucoheptonic, gluconic, glutamic, glycolic, glycollyarsanilic, hexylresorcinic, hydrabamic, hydrobromic, hydrochloric, hydroiodic, hydroxymaleic, hydroxynaphthoic, isethionic, lactic, lactobionic, lauryl sulfonic, maleic, malic, mandelic, meglumine, methane sulfonic, napsylic, nitric, oxalic, pamoic, pantothenic, phenylacetic, phosphoric, polygalacturonic, propionic, salicylic, stearic, subacetic, succinic, sulfamic, sulfanilic, sulfuric, tannic, tartaric, toluene sulfonic, and the commonly occurring amine acids, *e.g.,* glycine, alanine, phenylalanine, arginine, etc.

The pharmaceutically acceptable salts of the present invention can be synthesized from a parent compound that contains a basic or acidic moiety by conventional chemical methods. Generally, such salts can be prepared by reacting the free acid or base forms of these compounds with a stoichiometric amount of the appropriate base or acid in water or in an organic solvent, or in a mixture of the two. In one embodiment, non-aqueous media, for example ether, ethyl acetate, ethanol, isopropanol, or acetonitrile are useful for forming salts of the present compounds. Lists of suitable salts are found in Remington's Pharmaceutical Sciences, 18th ed. (Mack Publishing Company, 1990). For example, salts can include, but are not limited to, the hydrochloride and acetate salts of the aliphatic amine-containing, hydroxyl amine-containing, and imine-containing compounds of the present invention.

Additionally, the compounds of the present invention, for example, the salts of the compounds, can exist in either hydrated or unhydrated (the anhydrous) form or as solvates with other solvent molecules. Non-limiting examples of hydrates include monohydrates, dihydrates, etc. Non-limiting examples of solvates include ethanol solvates, acetone solvates, etc.

As used herein, "pharmaceutically acceptable esters" refer to derivatives of the disclosed compounds wherein the parent compound is modified by an alcohol ester of a carboxylic acid or a carboxylic acid ester of an alcohol. The compounds of the present invention can also be prepared as esters, for example pharmaceutically acceptable esters. For example a carboxylic acid function group in a compound can be converted to its corresponding ester, *e.g.,* a methyl, ethyl, or other ester. Also, an alcohol group in a compound can be converted to its corresponding ester, *e.g.,* an acetate, propionate, or other ester. In the present invention, particularly useful esters are the C₁-C₆ alkyl alcohol esters, *i.e.* the C₁-C₆ straight, branched, and cyclic alkyl esters, the phenyl ester, and the benzyl ester. Examples of these esters include the methyl ester, the ethyl ester, the n-propyl ester, the isopropyl ester, the n-butyl ester, and so forth.

As used herein, the term "unit dosage" or "unit dosage form", means a single dose of a pharmaceutical composition that is intended to be administered in its entirety. A unit dosage or unit dosage form is a convenient form for administering a premeasured amount of a drug active.

In the specification, the singular forms also include the plural, unless the context clearly dictates otherwise. Unless defined otherwise, all technical and scientific terms used herein have the same meaning as commonly understood by one of ordinary skill in the art to which this invention belongs. In the case of conflict, the present specification will control.

All percentages and ratios used herein, unless otherwise indicated, are by weight.

As used herein, the recitation of a numerical range for a variable is intended to convey that the invention may be practiced with the variable equal to any of the values within that range. Thus, for a variable which is inherently discrete, the variable can be equal to any integer value within the numerical range, including the end-points of the range. Similarly, for a variable which is inherently continuous, the variable can be equal to any real value within the numerical range, including the end-points of the range. As an example, and without limitation, a variable which is described as having values between 0 and 2 can take the values 0, 1 or 2 if the variable is inherently discrete, and can take the values 0.0, 0.1, 0.01, 0.001, or any other real values ≥ 0 and ≤ 2 if the variable is inherently continuous.

Throughout the description, where compositions are described as having, including, or comprising specific components, it is contemplated that compositions also consist essentially of, or consist of, the recited components. Similarly, where methods or processes are described as having, including, or comprising specific process steps, the processes also consist essentially of, or consist of, the recited processing steps. Further, it should be understood that the order of steps or order for performing certain actions is immaterial so long as the invention remains operable. Moreover, two or more steps or actions can be conducted simultaneously.

### 2. Compositions of the present invention

The compositions of the present invention comprise all or some of the following components. The compositions can be defined either prior to or after mixing of the components.

Suitable components are described in *e.g.,* Eds. R. C. Rowe, et al., Handbook of Pharmaceutical Excipients, Fifth Edition, Pharmaceutical Press (2006); Remington's Pharmaceutical Sciences, 18th ed. (Mack Publishing Company, 1990); and Remington: The Science and Practice of Pharmacy, 20th Edition, Baltimore, MD: Lippincott Williams & Wilkins, 2000. Even though a functional category can be provided for many of these carrier components, such a functional category is not intended to limit the function or scope of the component, as one of ordinary skill in the art will recognize that a component can belong to more than one functional category and that the level of a specific component and the presence of other components can affect the functional properties of a component.

### a. Quinolone Carboxylic Acid Derivative

The compositions of the present invent comprise a quinolone carboxylic acid derivative (alternatively known as, *inter alia,* a pyridonecarboxylic acid derivative or a pyridone carboxylic acid derivative), or a pharmaceutically acceptable salt thereof, as an antimicrobial compound, *i.e.* as the active pharmaceutical ingredient, or API, of the compositions of the present invention. The invention further provides methods for synthesizing any one of the compounds of the present invention. The invention also provides pharmaceutical compositions comprising an effective amount of one or more of the compounds of the present invention and a pharmaceutically acceptable carrier. The present invention further provides methods for making these compounds, carriers, and pharmaceutical compositions.

Quinolone carboxylic acid derivatives are described, including their syntheses, formulation, and use, in U.S. Patent No. 6,156,903, to Yazaki et al., issued December 5, 2000 and its certificates of correction of November 13, 2001 and December 11, 2001; U.S. Patent No. 6,133, 284, to Yazaki et al., issued October 17, 2000; U.S. Patent No. 5,998, 436, to Yazaki et al., issued December 7, 1999 and its certificates of correction of January 23, 2001, October 30, 2001, and December 17, 2002; PCT Application No. WO 2006/110815, to Abbott Laboratories, published October 19, 2006; PCT Application No. WO 2006/042034, to Abbott Laboratories, published April 20, 2006, PCT Application No. WO 2006/015194, to Abbott Laboratories, published February 9, 2006; PCT Application No. WO 01/34595, to Wakunaga Pharmaceutical Co., Ltd., published May 17, 2001; and PCT Application No. WO 97/11068, to Wakunaga Pharmaceutical Co., Ltd., published March 27, 1997. The present invention relates to a composition wherein said quinolone carboxylic acid corresponds to the compound (A): 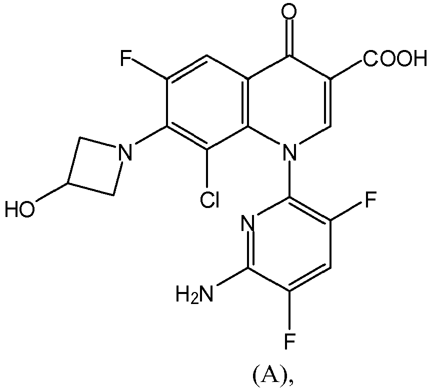 or a pharmaceutically acceptable salt or ester thereof. This foregoing quinolone carboxylic acid derivative, compound (A), is also known by the USAN, delafloxacin, the publicly disclosed code names RX-3341, ABT-492 and WQ 3034, and also by, inter alia, the chemical name 1-(6-amino-3,5-difluoro-2-pyridinyl)-8-chloro-6-fluoro-1,4-dihydro-7-(3-hydroxy-1-azetidinyl)-4-oxo-3-quinolinecarboxylic acid or 1-(6-amino-3,5-difluoro-2-pyridinyl)-8-chloro-6-fluoro-1,4-dihydro-7-(3-hydroxyazetidin-1-yl)-4-oxo-3-quinolinecarboxylic acid. This carboxylic acid form of the compound corresponds to the CAS Registry Number 189279-58-1. Furthermore, WO 2006/042034, cited above discloses the 1-deoxy-1-(methylamino)-D-glucitol salt of this compound, also known as D-glucitol, 1-deoxy-1-(methylamino)-, 1-(6-amino-3,5-difluoro-2-pyridinyl)-8-chloro-6-fluoro-1,4-dihydro-7-(3-hydroxy-1-azetidinyl)-4-oxo-3-quinolinecarboxylate , and the trihydrate of the 1-deoxy-1-(methylamino)-D-glucitol salt of this compound, also known as D-glucitol, 1-deoxy-1-(methylamino)-, 1-(6-amino-3,5-difluoro-2-pyridinyl)-8-chloro-6-fluoro-1,4-dihydro-7-(3-hydroxy-1-azetidinyl)-4-oxo-3-quinolinecarboxylate trihydrate. The 1-deoxy-1-(methylamino)-D-glucitol salt and the 1-deoxy-1-(methylamino)-D-glucitol salt trihydrate correspond to the CAS Registry Numbers 352458-37-8 and 883105-02-0, respectively. 1-Deoxy-1-(methylamino)-D-glucitol corresponds to the CAS Registry Number 6284-40-8. 1-Deoxy-1-(methylamino)-D-glucitol is also known by the name meglumine. D-glucitol, 1-deoxy-1-(methylamino)-, 1-(6-amino-3,5-difluoro-2-pyridinyl)-8-chloro-6-fluoro-1,4-dihydro-7-(3-hydroxy-1-azetidinyl)-4-oxo-3-quinolinecarboxylate, which is the meglumine salt of delafloxacin, is also known as delafloxacin meglumine. D-glucitol, 1-deoxy-1-(methylamino)-, 1-(6-amino-3,5-difluoro-2-pyridinyl)-8-chloro-6-fluoro-1,4-dihydro-7-(3-hydroxy-1-azetidinyl)-4-oxo-3-quinolinecarboxylate trihydrate, which is the trihydrate of the meglumine salt of delafloxacin, is also known as delafloxacin meglumine trihydrate. WO 2006/042034 also discloses a crystalline form of the 1-deoxy-1-(methylamino)-D-glucitol salt characterized when measured at about 25 °C with Cu-Kα radiation, by the x-ray powder diffraction pattern disclosed therein and a crystalline form of the 1-deoxy-1-(methylamino)-D-glucitol salt trihydrate when measured at about 25 °C with Cu-Ka Cu-Karadiation, by the x-ray powder diffraction pattern shown in FIG. 2 (see WO 2006/042034). These 1-deoxy-1-(methylamino)-D-glucitol salts are useful in the present invention. Also, *see* A.R. Haight et al. "Synthesis of the Quinolone ABT-492: Crystallizations for Optimal Processing", Organic Process Research & Development (2006), 10(4), 751-756.

Additionally other pharmaceutically acceptable salts of the forgoing compound, delafloxacin, include the potassium salt and the tris salt. Tris is a common abbreviation for tris(hydroxymethyl)aminomethane, which is known by the IUPAC name 2-Amino-2-hydroxymethyl-propane-1,3-diol.

In some embodiments, the quinolone carboxylic acid derivative comprises from about 0.01% to about 50% by weight of the composition. In further embodiments, the quinolone carboxylic acid derivative comprises from about 0.25% to about 20% by weight of the composition. In yet further embodiments, the quinolone carboxylic acid derivative comprises from about 0.5% to about 10% by weight of the composition. In yet further embodiments, the quinolone carboxylic acid derivative comprises from about 1% to about 5% by weight of the composition. The weight percentage of the quinolone carboxylic acid derivative is determined on an active weight basis of the parent compound. In other words, appropriate adjustments and calculations well known to one of ordinary skill in the art can be readily performed to determine the active weight basis. As a non-limiting example, if the parent free carboxylic acid of delafloxacin, *i.e.* 1-(6-amino-3,5-difluoro-2-pyridinyl)-8-chloro-6-fluoro-1,4-dihydro-7-(3-hydroxy-1-azetidinyl)-4-oxo-3-quinolinecarboxylic acid, is used, its weight would have to be adjusted if a salt such as the sodium salt were to be used, because the molecular weight of the compound would increase by about 21.9, although the amount of active compound delivered is the same.

In some embodiments, the quinolone carboxylic acid derivative is present in the pharmaceutical compositions described herein in the amount of from about 0.1 mg to about 1500 mg. In some embodiments, the quinolone carboxylic acid derivative is present in the pharmaceutical compositions described herein in the amount of from about 100 mg to about 750 mg. In some embodiments, the quinolone carboxylic acid derivative is present in the pharmaceutical compositions described herein in the amount of from about 250 to about 500 mg. In some embodiments, the quinolone carboxylic acid derivative is present in the pharmaceutical compositions described herein in the amount of about 300 mg. In some embodiments, the quinolone carboxylic acid derivative is present in the pharmaceutical compositions described herein in the amount of about 400 mg. In some embodiments, the quinolone carboxylic acid derivative is present in the pharmaceutical compositions described herein in the amount of about 450 mg. In some embodiments, the quinolone carboxylic acid derivative is present in the pharmaceutical compositions described herein in the amount of from about 0.1 to about 10 mg, from about 10 mg to about 20 mg, from about 20 mg to about 50 mg, from about 50 mg to about 100 mg, from about 100 mg to about 200 mg, from about 200 mg to about 500 mg, from about 500 mg to about 1000 mg or from about 1000 mg to about 1500 mg. In some embodiments, the quinolone carboxylic acid derivative is present in the pharmaceutical compositions described herein in the amount of about 5 mg, about 10 mg, about 15 mg, about 20 mg, about 25 mg, about 50 mg, about 75 mg, about 100 mg, about 125 mg, about 150 mg, about 175 mg, about 200 mg, about 225 mg, about 250 mg, about 275 mg, about 300 mg, about 325 mg, about 350 mg, about 375 mg, about 400 mg, about 425 mg, about 450 mg, about 475 mg, about 500 mg, about 525 mg, about 550 mg, about 575 mg, about 600 mg, about 625 mg, about 650 mg, about 675 mg about 700 mg, about 725 mg, about 750 mg, about 775 mg, about 800 mg, about 825 mg, about 850 mg, about 875 mg, about 900 mg, about 925 mg, about 950 mg, about 975 mg, about 1000 mg, about 1025 mg, about 1050, mg, about 1075 mg, about 1100 mg, about 1125 mg, about 1150 mg, about 1175 mg, about 1200 mg, about 1225 mg, about 1250 mg, about 1275 mg, about 1300 mg, about 1325 mg, about 1350 mg, about 1375 mg, about 1400 mg, about 1425 mg, about 1450 mg, about 1475 mg, or about 1500 mg.

The dose of the pharmaceutical active and mode of administration of the pharmaceutical composition will depend upon the intended patient or subject and the targeted microorganism, *e.g.,* the target bacterial organism.

As further described below, it is often advantageous to mill the pharmaceutical active to a small and uniform particle size, usually in the micron range, *i.e.* micronization. Milling can be performed using standard techniques well known to one of ordinary skill in the art. In one embodiment, useful particle size ranges for the pharmaceutical active are generally from about 0.01 microns to about 100 microns. In another embodiment, useful particle size ranges for the pharmaceutical active are from about 0.1 microns to about 20 microns. In another embodiment, useful particle size ranges for the pharmaceutical active are from about 0.5 microns to about 5 microns.

### b. Effervescent Agent(s)

The compositions of the present invention comprise an effervescent agent. Without being limited by theory, it is postulated that the effervescent agent can in certain instances help to drive the antimicrobial compound from the stomach into small intestine, and therefore into the blood stratum. Part of the reason for this is that in the highly acid environment of the stomach, the effervescent agent, which is typically the salt of a basic material or the mixture of a salt of a basic material and an acidic material or its salt, can react with the stomach acid to generate carbon dioxide bubbles, *i.e.* effervescence, which in turn can aid in tablet disintegration and movement or absorption of the antimicrobial agent from the stomach into the bloodstream. The net effect is believed to reduce the amount of free antimicrobial compound in the gastrointestinal tract, thus reducing the potential for undesirable gastrointestinal side effects or contributing to the development of antibiotic resistance. The invention relates to a pharmaceutical composition wherein said effervescent agent comprises sodium bicarbonate, sodium dihydrogen phosphate, and citric acid.

In some embodiments, the invention relates to a pharmaceutical composition wherein said effervescent agent comprises sodium bicarbonate, sodium dihydrogen phosphate monohydrate (also known as sodium phosphate monobasic monohydrate), and anhydrous citric acid.

In some embodiments, the effervescent agent is present in the pharmaceutical compositions described herein in the amount from about 0.1 to about 1500 mg. In some embodiments, the effervescent agent is present in the pharmaceutical compositions described herein in the amount from about 100 mg to about 750 mg. In some embodiments, the effervescent agent is present in the pharmaceutical compositions described herein in the amount from about 250 mg to about 500 mg. In some embodiments, the effervescent agent is present in the pharmaceutical compositions described herein in the amount from about 0.1 to about 10 mg, from about 10 mg to about 20 mg, from about 20 mg to about 50 mg, from about 50 mg to about 100 mg, from about 100 mg to about 200 mg, from about 200 mg to about 500 mg, from about 500 mg to about 1000 mg or from about 1000 mg to about 1500 mg. In some embodiments, the effervescent agent is present in the pharmaceutical compositions described herein in the amount of about 5 mg, about 10 mg, about 15 mg, about 20 mg, 25 mg, about 50 mg, about 75 mg, about 100 mg, about 125 mg, about 150 mg, about 175 mg, about 200 mg, about 225 mg, about 250 mg, about 275 mg, about 300 mg, about 325, about 350 mg, about 375 mg, about 400 mg, about 425 mg, about 450 mg, about 475 mg, about 500 mg, about 525 mg, about 550 mg, about 575 mg, about 600 mg, about 625 mg, about 650 mg, about 675 mg about 700 mg, about 725 mg, about 750 mg, about 775 mg, about 800 mg, about 825 mg, about 850 mg, about 875 mg, about 900 mg, about 925 mg, about 950 mg, about 975 mg, about 1000 mg, about 1025 mg, about 1050, mg, about 1075 mg, about 1100 mg, about 1125 mg, about 1150 mg, about 1175 mg, about 1200 mg, about 1225 mg, about 1250 mg, about 1275 mg, about 1300 mg, about 1325 mg, about 1350 mg, about 1375 mg, about 1400 mg, about 1425 mg, about 1450 mg, about 1475 mg, and about 1500 mg.

### c. Water

In one embodiment, the compositions of the present invention comprise from about 0.1 % to about 99.9% water, in further embodiments from about 1% to about 99% water, in yet further embodiments from about 5% to about 95% water, and in yet further embodiments from about 10% to about 90% water. In defining a composition, the amount of water can be designated as "q.s." or "Q.S.", which means as much as suffices, to provide a final composition or volume of 100%.

### d. Sugars and Sugar Alcohols

The compositions of the present invention, when further made into a lyophile, can further comprise a sugar, a sugar alcohol, or mixtures thereof. Without being limited by theory, these sugars and sugar alcohols are believed to aid in the formation of the lyophile during the lyophilization process. Typically, the lyophile is made by drying the composition under appropriate conditions, such as, for example, by freeze drying. Non-limiting examples of sugars include mannose, sucrose, dextrose, and mixtures thereof. Non-limiting examples of sugar alcohols useful herein include sorbitol, mannitol, xylitol and mixtures thereof.

In one embodiment, the compositions comprise from about 0.1% to about 50% of a sugar or sugar alcohol.

### e. Polyhydroxy Amine Compound

In one embodiment, the compositions of the present invention comprise a polyhydroxy amine compound. The polyhydroxy amine compound is separate from and does not encompass the polyhydroxy compound of the compositions of the present invention. The polyhydroxy amine compound is generally a C₃-C₈ straight, branched, or cyclic compound having 2 or more hydroxy substituents, and at least one amine (either substituted or unsubstituted) substituent.

In further embodiments the polyhydroxy amine compound is meglumine. Meglumine corresponds to CAS Registry Number 6284-40-8 and is also known as meglumine, USP; 1-Deoxy-1-(methylamino)-D-glucitol; N-Methyl-D-glucamine; Glucitol, 1-deoxy-1-(methylamino)-, D- (8C1); Sorbitol, 1-deoxy-1-methylamino- (6C1); 1-Deoxy-1-(methylamino)-D-glucitol; 1-Deoxy-1-methylaminosorbitol; D-(-)-N-Methylglucamine; Meglumin; Methylglucamin; Methylglucamine; N-Methyl-D(-)-glucamine; N-Methyl-D-glucamine; N-Methylglucamine; N-Methylsorbitylamine; NSC 52907; NSC 7391. It also has the CA Index Name D-Glucitol, 1-deoxy-1-(methylamino)- (9C1). A chemical formula for meglumine is as follows:

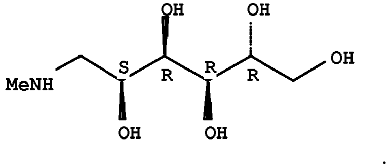

In one embodiment, the polyhydroxy amine compound comprises from about 0.1% to about 50% by weight of the composition. In further embodiments, the polyhydroxy amine compound comprises from about 0.25% to about 20% by weight of the composition. In yet further embodiments, the polyhydroxy amine compound comprises from about 0.5% to about 10% by weight of the composition. In yet further embodiments, the polyhydroxy amine compound comprises from about 1% to about 5% by weight of the composition.

### f. Chelating Agents

The compositions of the present invention can further comprise a chelating agent. It is to be understood that this chelating agent is defined herein as being separate from and does not include the already described effervescent agent, which can also have chelating properties. The chelating agent is also defined herein as excluding the cyclodextrin, the polyhydroxy amine compound, or any of the other components described herein, even though the cyclodextrin, the polyhydroxy amine compound, or other components described herein can also have chelating properties. An example of a chelating agent useful herein is EDTA, also known as ethylenediaminetetraacetic acid, or a salt thereof. Useful salts include, for example, a sodium salt, a potassium salt, a calcium salt, a magnesium salt, and mixtures of these salts. An example of a mixture of salts or a mixed salt is the monosodium monocalcium salt of EDTA. It is found that the disodium salt of EDTA, also known as disodium EDTA, is useful herein. For convenience, the disodium EDTA can first be separately prepared as an aqueous solution for use in formulating the compositions of the present invention.

In one embodiment, the disodium EDTA comprises from about 0.0010% to about 0.10% by weight of the composition. In further embodiments, the disodium EDTA comprises from about 0.0050% to about 0.050% by weight of the composition. In yet further embodiments, the disodium EDTA comprises from about 0.010% to about 0.020% by weight of the composition. In other embodiments the disodium EDTA comprises about 0.010% of the composition, or about 0.011% of the composition, or about 0.012% of the composition, or about 0.013% of the composition, or about 0.014% of the composition, or about 0.015% of the composition, or about 0.016% of the composition, or about 0.017% of the composition, or about 0.018% of the composition, or about 0.019% of the composition, or about 0.020% of the composition. These weight percentages of the disodium EDTA described herein are on the basis of the ethylenediaminetetraacetic acid.

### g. pH Modifiers and pH of the Compositions

The compositions of the present invention can further comprise various materials for modifying or adjusting the pH of the composition. Such materials include acids, bases, buffer systems, etc. Non-limiting examples of such pH modifiers include, for example, hydrochloric acid and sodium hydroxide. Examples of other useful materials include triethanolamine, sodium carbonate, and lysine. Furthermore, the polyhydroxy amine compound, such as described above, can be used as a pH modifier. More specifically, the polyhydroxy amine compound, meglumine, can be used as a pH modifier. In some embodiments, the pH modifier is present in the composition in an amount of 1-5 mg, 5-50 mg, 50-100 mg, 100-200 mg, or 200-500 mg.

The compositions of the present invention should have a pH so that the composition is suitable for administration to a patient or subject. The compositions have a pH from about pH 7 to about pH 11. In further embodiments, the compositions have a pH from about pH 8 to about pH 10. In further embodiments, the compositions have a pH from about pH 8.5 to about pH 9.5. In further embodiments, the compositions have a pH from about pH 8.8 to about pH 9.2. In further embodiments, the compositions have a pH of about 9.0.

### h. Additional Components

Pharmaceutical compositions of the present invention comprise an effective amount of a quinolone carboxylic acid derivative or a pharmaceutically acceptable salt thereof and an effervescent agent, or one or more additional agents dissolved or dispersed in a pharmaceutically acceptable carrier. The phrases "pharmaceutical or pharmacologically acceptable" refers to molecular entities and compositions that do not produce an adverse, allergic or other untoward reaction when administered to an animal, such as, for example, a human, as appropriate. The preparation of a pharmaceutical composition that contains an effective amount of a quinolone carboxylic acid derivative or a pharmaceutically acceptable salt thereof and an effervescent agent will be known to those of skill in the art in light of the present disclosure, as exemplified by Remington's Pharmaceutical Sciences, 18th ed. Mack Printing Company, 1990. Moreover, for animal (*e.g.*, human) administration, it will be understood that preparations should meet sterility, pyrogenicity, general safety and purity standards as required by Food and Drug Administration's Office of Biological Standards.

The compositions of the present invention can further comprise one or more additional components selected from a wide variety of excipients known in the pharmaceutical formulation art. According to the desired properties of the tablet or capsule, any number of ingredients can be selected, alone or in combination, based upon their known uses in preparing the compositions of the present invention. As used herein, "pharmaceutically acceptable carrier" includes any and all solvents, dispersion media, coatings, surfactants, antioxidants, preservatives (*e.g.*, antibacterial agents, antifungal agents), isotonic agents, absorption delaying agents, salts, preservatives, drugs, drug stabilizers, gels, binders, excipients, disintegration agents, lubricants, sweetening agents, flavoring agents, dyes, such like materials and combinations thereof, as would be known to one of ordinary skill in the art (see, for example, Remington's Pharmaceutical Sciences, 18th ed. Mack Printing Company, 1990, pp. 1289 1329). Except insofar as any conventional carrier is incompatible with the active ingredient, its use in the therapeutic or pharmaceutical compositions is contemplated. Such ingredients include, but are not limited to solvents (*e.g.,* ethanol), dispersion media, coatings (*e.g.*, enteric polymers), isotonic agents, absorption delaying agents, salts, drugs, drug stabilizers, gels, plasticizers, binders, excipients, disintegration agents, lubricants, sweetening agents, flavoring agents, dyes, colorants; waxes, gelatin; preservatives (*e.g.,* antibacterial agents and antifungal agents such as methyl paraben, sodium benzoate, and potassium benzoate); antioxidants (*e.g.,* butylated hydroxyanisole ("BHA"), butylated hydroxytoluene ("BHT"), and vitamin E and vitamin E esters such as tocopherol acetate); surfactants; UV-absorbers, such like materials and combinations thereof. Exemplary cellulose ethers include hydroxyalkyl celluloses and carboxyalkyl celluloses. Exemplary cellulose ethers include hydroxyethyl celluloses, hydroxypropyl celluloses, hydroxypropylmethyl-celluloses, carboxy methylcelluloses, and mixtures thereof.

Any of the excipients can be present in the formulation in an amount of about 0 mg to about 1500 mg. In some embodiments, an excipient is present in the formulation in an amount of about 0 mg to about 5 mg, about 5 mg to about 10 mg, about 10 mg to about 20 mg, about 20 mg to about 50 mg, about 50 mg to about 100 mg, about 100 mg to about 200 mg, about 200 mg to about 500 mg, about 500 mg to about 800 mg, about 800 mg to about 1000 mg or about 1000 mg to about 1500 mg.

In some embodiments, microcrystalline cellulose is present in the formulation in an amount of about 0 mg to about 200 mg or about 200 mg to about 500 mg.

In some embodiments, water is present in the formulation in an amount of about 0 mg to about 500 mg.

In some embodiments, a binder is present in the formulation in an amount of about 0 mg to about 100 mg or about 0 to about 200 mg.

In some embodiments, a disintegrant is present in the formulation in an amount of about 0 mg to about 100 mg or about 0 mg to about 200 mg.

In some embodiments, a film coating is present in a tablet in an amount of about 0 mg to about 20 mg or about 0 mg to about 200 mg.

In some embodiments, a lubricant is present in the formulation in an amount of about 0.1 mg to about 20 mg. In further embodiments, a lubricant is present in the formulation in an amount of about 0.5 mg to about 10 mg.

In some embodiments, an enteric coating is present in a tablet in an amount of about 0 mg to about 20 mg or about 0 mg to about 200 mg.

In some embodiments, a plasticizer is present in a tablet in an amount of about 1 mg to about 30 mg.

In one embodiment, the compositions of the present invention comprise a carrier. The carrier can be a dextrose solution or saline, at a pharmaceutically acceptable concentration. The composition comprising a carrier can be administered to a patient via an i.v. bag.

### 3. Processing

The compositions of the present invention are made using conventional equipment and mixing techniques, such as dry granulation or wet granulation. Examples of techniques used to make the compositions described herein are provided in Remington's Pharmaceutical Sciences, 17th edition, 1985, Editor: Alfonso R. Gennaro, Mack Publishing Company, Easton, PA 18 042.

### 4. Gastrointestinal Tolerability

The compositions of the present invention have improved gastrointestinal tolerability. Also, the compositions of the present invention reduce the potential for gastrointestinal side effects. Non-limiting gastrointestinal side effects include diarrhea, flatulence, nausea, vomiting, abdominal pain, dyspepsia, belching, bloating, gastritis, and general abdominal discomfort. The gastrointestinal tolerability of the compositions of the present invention can be evaluated using various models as described in the Examples below.

### 5. Doses and Methods of Treating, Preventing, or Reducing the Risk of Infections

In some embodiments, the compositions disclosed herein are useful in treating microbial infections (*e.g.*, bacterial infections).

The compositions of the present invention are useful for treating, preventing or reducing the risk of a microbial infection, *e.g.,* a skin infection, a skin and skin structure infections such as a complicated skin and skin structure infection (cSSSI), an uncomplicated skin and skin structure infection (uSSSI) and an acute bacterial skin and skin structure infection (ABSSSI), pneumonia and other respiratory tract infections such as a community respiratory-tract infection, a nosocomial (hospital-acquired) pneumonia, a community acquired pneumonia, a hospital acquired community pneumonia and a post-viral pneumonia, an abdominal infection, a urinary tract infection, bacteremia, septicemia, endocarditis, an atrio-ventricular shunt infection, a vascular access infection, meningitis, an infection due to surgical or invasive medical procedures, a peritoneal infection, a bone infection, a joint infection, a methicillin-resistant *Staphylococcus aureus* (MRSA) infection, a vancomycin-resistant *Enterococci* infection, a linezolid-resistant organism infection, tuberculosis, a quinolone resistant Gram-positive infection, a ciprofloxacin resistant methicillin resistant (MRSA) infection, bronchitis, uncomplicated gonorrhea, and a multi-drug resistant (MDR) Gram-negative infection.

The dose of active compound and mode of administration, *e.g.,* injection, intravenous drip, etc. will depend upon the intended patient or subject and the targeted microorganism, *e.g.,* the target bacterial organism. Dosing strategies are disclosed in L.S. Goodman, et al., The Pharmacological Basis of Therapeutics, 201-26 (5th ed. 1975).

Compositions can be formulated in unit dosage form for ease of administration and uniformity of dosage. Unit dosage form refers to physically discrete units suited as unitary dosages for the subject to be treated; each unit containing a predetermined quantity of active compound calculated to produce the desired therapeutic effect in association with the required pharmaceutical carrier. The specification for the unit dosage forms of the invention are dictated by and directly dependent on the unique characteristics of the active compound and the therapeutic effect to be achieved, and the limitations inherent in the art of compounding such an active compound for the treatment of individuals.

Compositions of the present invention are made in the form of a dry mixture, an aqueous solution, an aqueous suspension, a non-aqueous solution, a non-aqueous suspension, or an emulsion. The pharmaceutical compositions are made in unit dosage forms, which may further be in the form of a tablet or a capsule. The tablet may be a bilayer tablet comprising more than one layer. In one embodiment, the bilayer tablet comprises a first and a second layer. In some embodiments, the first layer comprises the quinolone carboxylic acid derivative or a pharmaceutically acceptable salt or ester thereof and the second layer comprises the aluminum compound. In some embodiments, the second layer in the bilayer tablet is a delayed or sustained or controlled release layer, wherein the second layer is released in the gastrointestinal tract downstream from the stomach. Alternatively, the quinolone carboxylic acid derivative or a pharmaceutically acceptable salt or ester thereof and the aluminum compound are administered as separate unit dosage forms. In some embodiments, when the quinolone carboxylic acid derivative or a pharmaceutically acceptable salt or ester thereof and the aluminum compound are administered as separate unit dosage forms, the quinolone carboxylic acid derivative or a pharmaceutically acceptable salt or ester thereof is administered first and the aluminum compound is administered second and within two hours of the quinolone carboxylic acid derivative. In yet other embodiments, the quinolone carboxylic acid derivative or a pharmaceutically acceptable salt or ester and the aluminum compound are concurrently administered as separate unit dosage forms.

In conjunction with the methods of the present invention, pharmacogenomics (*i.e.* the study of the relationship between an individual's genotype and that individual's response to a foreign compound or drug) can be considered. Differences in metabolism of therapeutics can lead to severe toxicity or therapeutic failure by altering the relation between dose and blood concentration of the pharmacologically active drug. Thus, a physician or clinician can consider applying knowledge obtained in relevant pharmacogenomics studies in determining whether to administer a drug as well as tailoring the dosage and/or therapeutic regimen of treatment with the drug.

Generally, an effective amount of dosage of active compound will be in the range of from about 0.1 to about 100 mg/kg of body weight/day. In one embodiment, the amount will be from about 1.0 to about 50 mg/kg of body weight/day. The amount administered will also likely depend on such variables as the overall health status of the patient, the relative biological efficacy of the compound delivered, the formulation of the drug, the presence and types of excipients in the formulation, the route of administration, and the infection to be treated, prevented, or reducing the risk of. Also, it is to be understood that the initial dosage administered can be increased beyond the above upper level in order to rapidly achieve the desired blood-level or tissue level, or the initial dosage can be smaller than the optimum.

In some embodiments, the compositions disclosed herein comprise a dose of active compound of from about 0.1 to about 1500 mg per dose. In some embodiments, the compositions disclosed herein comprise a dose of active compound of about 100 mg to about 750 mg per dose. In some embodiments, the compositions disclosed herein comprise a dose of active compound of about 250 mg to about 500 mg per dose. In some embodiments, the compositions disclosed herein comprise a dose of active compound of about 300 mg per dose. In some embodiments, the compositions disclosed herein comprise a dose of active compound of about 400 mg per dose. In some embodiments, the compositions disclosed herein comprise a dose of active compound of about 450 mg per dose. In some embodiments, the compositions disclosed herein comprise a dose of active compound of about 0.1 to about 10 mg per dose, about 10 mg to about 20 mg per dose, about 20 mg to about 50 mg per dose, about 50 mg to about 100 mg per dose, about 100 mg to about 200 mg per dose, about 200 mg to about 500 mg per dose, about 500 mg to about 1000 mg per dose or about 1000 mg to about 1500 mg per dose. Non-limiting examples of doses, which can be formulated as a unit dose for convenient administration to a patient include: about 5 mg, about 10 mg, about 15 mg, about 20 mg, about 25 mg, about 50 mg, about 75 mg, about 100 mg, about 125 mg, about 150 mg, about 175 mg, about 200 mg, about 225 mg, about 250 mg, about 275 mg, about 300 mg, about 325 mg, about 350 mg, about 375 mg, about 400 mg, about 425 mg, about 450 mg, about 475 mg, about 500 mg, about 525 mg, about 550 mg, about 575 mg, about 600 mg, about 625 mg, about 650 mg, about 675 mg about 700 mg, about 725 mg, about 750 mg, about 775 mg, about 800 mg, about 825 mg, about 850 mg, about 875 mg, about 900 mg, about 925 mg, about 950 mg, about 975 mg, about 1000 mg, about 1025 mg, about 1050, mg, about 1075 mg, about 1100 mg, about 1125 mg, about 1150 mg, about 1175 mg, about 1200 mg, about 1225 mg, about 1250 mg, about 1275 mg, about 1300 mg, about 1325 mg, about 1350 mg, about 1375 mg, about 1400 mg, about 1425 mg, about 1450 mg, about 1475 mg, and about 1500 mg. The foregoing doses are useful for administering the compounds of the present invention according to the methods of the present invention. The foregoing doses are particularly useful for administering the quinolone carboxylic acid derivatives of the present invention, particularly the compound known by the name delafloxacin and pharmaceutically acceptable salts, esters and prodrugs thereof.

As is understood by one of ordinary skill in the art, generally, when dosages are described for a pharmaceutical active, the dosage is given on the basis of the parent or active moiety. Therefore, if a salt, hydrate, or another form of the parent or active moiety is used, a corresponding adjustment in the weight of the compound is made, although the dose is still referred to on the basis of the parent or active moiety delivered. As a non-limiting example, if the parent or active moiety of interest is a monocarboxylic acid having a molecular weight of 250, and if the monosodium salt of the acid is desired to be delivered to be delivered at the same dosage, then an adjustment is made recognizing that the monosodium salt would have a molecular weight of approximately 272 (*i.e.* minus 1H or 1.008 atomic mass units and plus 1 Na or 22.99 atomic mass units). Therefore, a 250 mg dosage of the parent or active compound would correspond to about 272 mg of the monosodium salt, which would also deliver 250 mg of the parent or active compound. Said another way, about 272 mg of the monosodium salt would be equivalent to a 250 mg dosage of the parent or active compound.

In one embodiment, compositions of the invention are useful in the manufacture of a medicament for treating, preventing or reducing the risk of infection in a patient in need thereof. In another embodiment, delafloxacin, or a pharmaceutically acceptable salt thereof, is useful in the manufacture of a medicament for treating, preventing or reducing the risk of infection in a patient in need thereof. Such infections can be due to, *e.g.,* a skin infection, a skin and skin structure infections such as a complicated skin and skin structure infection (cSSSI), an uncomplicated skin and skin structure infection (uSSSI) and an acute bacterial skin and skin structure infection (ABSSSI), pneumonia and other respiratory tract infections such as a community respiratory-tract infection, a nosocomial (hospital-acquired) pneumonia, a community acquired pneumonia, a hospital acquired community pneumonia and a post-viral pneumonia, an abdominal infection, a urinary tract infection, bacteremia, septicemia, endocarditis, an atrio-ventricular shunt infection, a vascular access infection, meningitis, an infection due to surgical or invasive medical procedures, a peritoneal infection, a bone infection, a joint infection, a methicillin-resistant *Staphylococcus aureus* (MRSA) infection, a vancomycin-resistant *Enterococci* infection, a linezolid-resistant organism infection, tuberculosis, a quinolone resistant Gram-positive infection, a ciprofloxacin resistant methicillin resistant (MRSA) infection, bronchitis, uncomplicated gonorrhea, and a multi-drug resistant (MDR) Gram-negative infection.

In some embodiments, the pharmaceutical composition comprises: from about 0.01% to about 80 % by weight of a quinolone carboxylic acid derivative, or a pharmaceutically acceptable salt thereof, as compared to the total weight of the composition on an acid basis; and from about 0.1% to about 50% by weight of an effervescent agent, as compared to the total weight of the composition.

In some embodiments, the pharmaceutical composition comprises: from about 1% to about 60 % by weight of a quinolone carboxylic acid derivative, or a pharmaceutically acceptable salt thereof, as compared to the total weight of the composition on an acid basis; and from about 0.1% to about 50% by weight of an effervescent agent, as compared to the total weight of the composition.

In some embodiments, the pharmaceutical composition comprises: from about 10% to about 50 % by weight of a quinolone carboxylic acid derivative, or a pharmaceutically acceptable salt thereof, as compared to the total weight of the composition on an acid basis; and from about 0.1% to about 50% by weight of an effervescent agent, as compared to the total weight of the composition.

In some embodiments , the pharmaceutical composition comprising: from about 0.01% to about 80% by weight of a quinolone carboxylic acid derivative, or a pharmaceutically acceptable salt thereof, as compared to the total weight of the composition on an acid basis; from about 0.1% to about 50% by weight of meglumine, as compared to the total weight of the composition; and from about 0.1% to about 50% by weight of an effervescent agent, as compared to the total weight of the composition.

In some embodiments , the pharmaceutical composition comprising: from about 0.01% to about 80% by weight of a quinolone carboxylic acid derivative, or a pharmaceutically acceptable salt thereof, as compared to the total weight of the composition on an acid basis; from about 1% to about 40% by weight of meglumine, as compared to the total weight of the composition; and from about 0.1% to about 50% by weight of an effervescent agent, as compared to the total weight of the composition.

In some embodiments , the pharmaceutical composition comprising: from about 0.01% to about 80% by weight of a quinolone carboxylic acid derivative, or a pharmaceutically acceptable salt thereof, as compared to the total weight of the composition on an acid basis; from about 10% to about 30% by weight of meglumine, as compared to the total weight of the composition; and from about 0.1% to about 50% by weight of an effervescent agent, as compared to the total weight of the composition.

Using delafloxacin as example, in some embodiments, an example of a composition useful in the methods of the present invention contains about 300 mg of delafloxacin, or a pharmaceutically acceptable salt thereof. In further embodiments, an example of a composition useful in the methods of the present invention contains about 400 mg of delafloxacin, or a pharmaceutically acceptable salt thereof. In further embodiments, an example of a composition useful in the methods of the present invention contains about 450 mg of delafloxacin, or a pharmaceutically acceptable salt thereof.

### 6. Examples

The following examples further describe and demonstrate embodiments within the scope of the present invention. The examples are given solely for the purpose of illustration and are not to be construed as limitations of the present invention.

Ingredients are identified by chemical, USP, or CTFA name.

The following formulations are preparing using mixing techniques and equipment familiar to one of ordinary skill in the art.

The disclosed formulations are useful for oral administration to a patient for treating, preventing, or reducing the risk of a microbial infection, *e.g.,* a skin infection, a skin and skin structure infections such as a complicated skin and skin structure infection (cSSSI), an uncomplicated skin and skin structure infection (uSSSI) and an acute bacterial skin and skin structure infection (ABSSSI), pneumonia and other respiratory tract infections such as a community respiratory-tract infection, a nosocomial (hospital-acquired) pneumonia, a community acquired pneumonia, a hospital acquired community pneumonia and a post-viral pneumonia, an abdominal infection, a urinary tract infection, bacteremia, septicemia, endocarditis, an atrio-ventricular shunt infection, a vascular access infection, meningitis, an infection due to surgical or invasive medical procedures, a peritoneal infection, a bone infection, a joint infection, a methicillin-resistant *Staphylococcus aureus* (MRSA) infection, a vancomycin-resistant *Enterococci* infection, a linezolid-resistant organism infection, tuberculosis, a quinolone resistant Gram-positive infection, a ciprofloxacin resistant methicillin resistant (MRSA) infection, bronchitis, uncomplicated gonorrhea, and a multi-drug resistant (MDR) Gram-negative infection. More specifically, the disclosed formulations are useful for reducing the risk of or preventing infection due to a surgical or invasive medical procedure to be performed upon the patient, and in such case, the formulation can be administered just prior to or up to about 1 hour prior to the surgical or invasive medical procedure.

### EXAMPLE 1

The following is a sample formulation comprising the combination of delafloxacin and an effervescent agent. The bilayer tablet of this example was made using standard formulation and tableting techniques. Table 1 provides a Quantitative Composition of 400 mg Delafloxacin Bilayer Tablet.

**TABLE 1**

| **Ingredient** | **mg/tablet** | **% w/w** |
|---|---|---|
| Drug Layer, Intra-granular (Wet Granulation) | | |
| Delafloxacin (Meglumine salt)⁽¹⁾ | 577.2 | 41.23 |
| Cellulose, Microcrystalline, Avicel PH 101⁽²⁾ | 287.8 | 20.56 |
| Povidone | 30. | 2.1 |
| Crospovidone, NF, Kollidon CL | 50. | 3.6 |
| Water, USP ⁽³⁾ | 170. | N/A |
| Water, USP ⁽⁴⁾ | q.s. (Up to 100.) | N/A |
| Drug Layer, Extra-granular | | |
| Crospovidone, NF, Kollidon CL | 50. | 3.6 |
| Magnesium stearate, NF | 5.0 | 0.36 |
| **Drug Layer Total** | **1000** | **71.5** |
| | | |
| Buffering Layer, Intra-granular (Dry Granulation) | | |
| Sodium bicarbonate, powder, USP | 140. | 10.0 |
| Sodium phosphate monobasic, monohydrate, USP | 5.5 | 0.39 |
| Citric acid, Anhydrous, powder, USP | 5.5 | 0.39 |
| Cellulose, Microcrystalline, Avicel PH 101 | 247 | 17.6 |
| Magnesium stearate, NF | 0.8 | 0.06 |
| Buffering Layer, Extra-granular | | |
| Magnesium stearate, NF | 1.2 | 0.086 |
| **Buffering Layer Total** | **400** | **28.5** |
| **Total for Bilayer Tablet** | **1400** | **100** |

(1) Equivalent to 400 mg free acid (based on 69.3% salt conversion). The actual values may vary slightly depending on the purity and residue solvent content from the certificate of analysis (CoA).
(2) The exact amount will be adjusted based on the purity of API.
(3) For binder solution (PVP solution) preparation, and granulation. Removed during drying process.
(4) Maximum additional water for granulation, removed during drying process.

Table 2 provides a simplified Quantitative Composition of 400 mg Delafloxacin Bilayer Tablet, showing total amounts of each ingredient (*i.e.* where the components are not separated by granulation or layer).

**TABLE 2**

| **Ingredient** | **mg/tablet** | **% w/w** |
|---|---|---|
| Delafloxacin (Meglumine salt)⁽¹⁾ | 577.2 | 41.23 |
| Cellulose, Microcrystalline, Avicel PH 101⁽²⁾ | 535 | 38.2 |
| Povidone | 30. | 2.1 |
| Crospovidone, NF, Kollidon CL | 100. | 7.14 |
| Sodium bicarbonate, powder, USP | 140. | 10.0 |
| Sodium phosphate monobasic, monohydrate, USP | 5.5 | 0.39 |
| Citric acid, Anhydrous, powder, USP | 5.5 | 0.39 |
| Magnesium stearate, NF | 7.0 | 0.50 |
| **Total** | **1400** | **100** |

| | | |
|---|---|---|
| (1) Equivalent to 400 mg free acid (based on 69.3% salt conversion). The actual values may vary slightly depending on the purity and residue solvent content from the certificate of analysis (CoA). (2) The exact amount will be adjusted based on the purity of API. | | |

The foregoing composition is useful for oral administration to a patient for treating, preventing, or reducing the risk of a microbial infection.

### EXAMPLE 2

The following is a sample formulation comprising the combination of delafloxacin and an effervescent agent. The single layer tablet of this example was made using standard formulation and tableting techniques. Table 3 provides a Quantitative Composition of 400 mg Delafloxacin Single Layer Tablet.

**TABLE 3**

| **Ingredient** | **mg/tablet** | **% w/w** |
|---|---|---|
| Drug Granulation | | |
| Delafloxacin (Meglumine salt)⁽¹⁾ | 577.2 | 46.18 |
| Cellulose, Microcrystalline, PH 101⁽²⁾ | 262.8 | 21.02 |
| Povidone | 30.0 | 2.40 |
| Crospovidone, NF | 75.0 | 6.00 |
| Water, USP⁽³⁾ | 170. | N/A |
| Water, USP⁽⁴⁾ | q.s. (Up to 1 C 0 ) | N/A |
| **Total Drug Granule** | 945 | 75.6 |
| | | |

| Buffering Blend | | |
|---|---|---|
| Sodium bicarbonate | 140. | 11.2 |
| Sodium phosphate monobasic, monohydrate | 5.5 | 0.44 |
| Citric acid, Anhydrous | 5.5 | 0.44 |
| Crospovidone, NF | 25.0 | 2.00 |
| Cellulose, Microcrystalline, PH 101 | 124 | 9.92 |
| **Total Buffering Blend** | 300 | 24 |
| | | |
| Magnesium stearate | 5.0 | 0.40 |
| **Final Tablet Weight** | **1250** | **100** |

| | | |
|---|---|---|
| (1) Equivalent to 400 mg free acid (based on 69.3% salt conversion). The actual values may vary slightly depending on the purity and residue solvent content from the certificate of analysis (CoA). (2) The exact amount will be adjusted based on the purity of API. (3) For binder solution (PVP solution) preparation and granulation. Removed during drying process. (4) Maximum additional water for granulation, removed during drying process. | | |

Table 4 provides a simplified Quantitative Composition of 400 mg Delafloxacin Single Layer Tablet, showing total amounts of each ingredient (*i.e.* where the components are not separated by granulation).

**TABLE 4**

| **Ingredient** | **mg/tablet** | **% w/w** |
|---|---|---|
| Delafloxacin (Meglumine salt)⁽¹⁾ | 577.2 | 46.18 |
| Cellulose, Microcrystalline, PH101⁽²⁾ | 387 | 31.0 |
| Povidone | 30.0 | 2.40 |
| Crospovidone, NF | 100. | 8.00 |
| Sodium bicarbonate | 140. | 11.2 |
| Sodium phosphate monobasic, monohydrate | 5.5 | 0.44 |
| Citric acid, Anhydrous | 5.5 | 0.44 |
| Magnesium stearate | 5.0 | 0.40 |
| **Total** | **1250** | **100** |

| | | |
|---|---|---|
| (1) The amount of delafloxacin is based on a theoretical potency of 100% as free acid (based on 69.3% salt conversion). The exact amount may vary slightly depending on the purity and residue solvent content from the certificate of analysis (CoA). (2) The exact amount will be adjusted based on the purity of API. | | |

The foregoing composition is useful for oral administration to a patient for treating, preventing, or reducing the risk of a microbial infection.

### EXAMPLE 3

The following is a sample formulation comprising the combination of delafloxacin and an effervescent agent. The single layer tablet of this example was made using standard formulation and tableting techniques. The 450 mg single layer tablet improves tablet dissolution and reduces overall tablet weight compared to a 400 mg bilayer tablet. Table 5 provides a Quantitative Composition of 450 mg Delafloxacin Single Layer Tablet.

**TABLE 5**

| **Ingredient** | **mg/tablet (450 mg)** | **% w/w** |
|---|---|---|
| Drug Granulation | | |
| Delafloxacin (Meglumine salt)⁽¹⁾ | 649.4 | 47.40 |
| Microcrystalline Cellulose, PH 101⁽²⁾ | 295.8 | 21.59 |
| Povidone | 33.8 | 2.47 |
| Crospovidone | 84.0 | 6.13 |
| Water, USP ⁽³⁾ | 191.3 | NA |
| Water, USP ⁽⁴⁾ | 60. | NA |
| **Total drug granule** | **1063** | **77.6** |

| Extragranular | | |
|---|---|---|
| Sodium bicarbonate | 140. | 10.2 |
| Sodium phosphate monobasic, monohydrate | 5.5 | 0.40 |
| Citric acid, Anhydrous | 5.5 | 0.40 |
| Crospovidone | 25.0 | 1.82 |
| Microcrystalline Cellulose, PH 101 | 121.0 | 8.83 |
| Magnesium stearate | 10. | 0.73 |
| **Total extragranular** | **307** | **22.4** |
| **FINAL TABLET** | **1370** | **100** |

| | | |
|---|---|---|
| (1) The amount of delafloxacin is based on a theoretical potency of 100% as free acid (based on 69.3% salt conversion). The exact amount may vary slightly depending on the purity and residue solvent content from the certificate of analysis (CoA). (2) The exact amount of microcrystalline cellulose will be adjusted based on the purity of API. (3) For binder solution preparation, and granulation. Removed during drying process. (4) Target additional water for granulation, final amount depending on granulation end point with upper limit of 100 mg. This water is removed during drying process. | | |

Table 6 provides a simplified Quantitative Composition of 450 mg Delafloxacin Single Layer Tablet, showing total amounts of each ingredient (*i.e.* where the components are not separated by granulation).

**TABLE 6**

| **Ingredient** | **mg/tablet** | **% w/w** |
|---|---|---|
| Delafloxacin (Meglumine salt)⁽¹⁾ | 649.4 | 47.40 |
| Microcrystalline Cellulose, PH 101⁽²⁾ | 416.8 | 30.42 |
| Povidone | 33.8 | 2.47 |
| Crospovidone | 109 | 7.96 |
| Sodium bicarbonate | 140. | 10.2 |
| Sodium phosphate monobasic, monohydrate | 5.5 | 0.40 |
| Citric acid, Anhydrous | 5.5 | 0.40 |
| Magnesium stearate | 10. | 0.73 |
| **Total** | **1370** | **100** |

| | | |
|---|---|---|
| (1) The amount of delafloxacin is based on a theoretical potency of 100% as free acid (based on 69.3% salt conversion). The exact amount may vary slightly depending on the purity and residue solvent content from the certificate of analysis (CoA). (2) The exact amount of microcrystalline cellulose will be adjusted based on the purity of API. | | |

The foregoing composition is useful for oral administration to a patient for treating, preventing, or reducing the risk of a microbial infection. In particular, this composition was successful at achieving an AUC nearly identical to that of a 300 mg intravenous formulation of delafloxacin meglumine. Notably, this composition includes an increased drug content as compared with the Bilayer Tablet composition of Example 1, but with reduced tablet weight.

### EXAMPLE 4

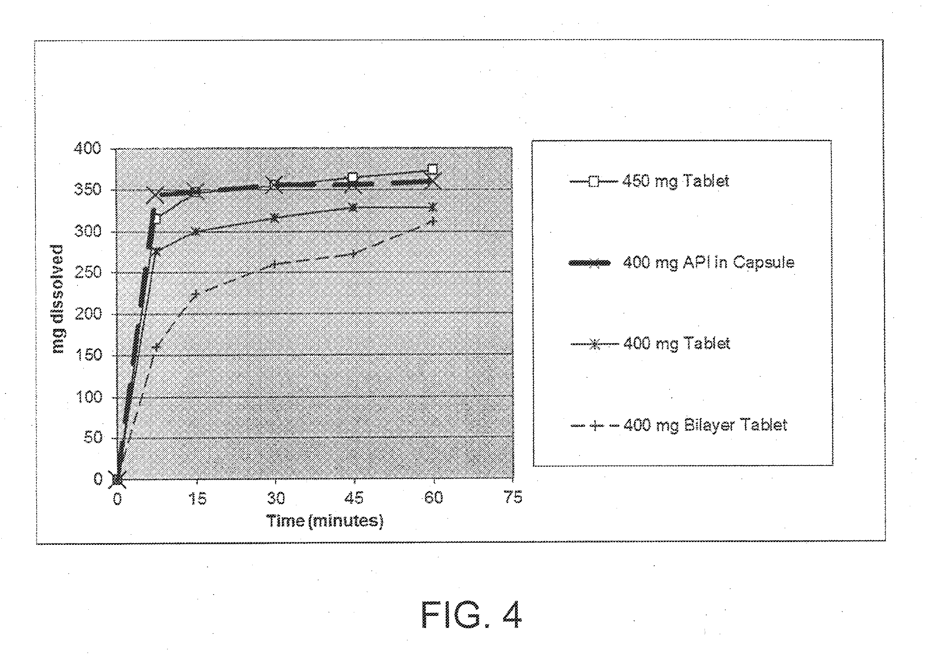

The results of a dissolution study performed by utilizing USP dissolution Apparatus 2 at a paddle speed of 50 rpm, in 900 mL of 50mM Sodium Phosphate Buffer with 30 mM hexadecyltrimethylammonium bromide (HTAB), pH 6.8 as dissolution medium are shown in FIG. 4. The testing temperature was 37.0 ± 0.5 °C. The samples were pulled at 7.5, 15, 30, 45, 60 minutes and every additional 15 minutes to infinity at a higher paddle speed of 250 rpm. Samples were analyzed by HPLC and the amount of drug released were measured and calculated against the label claimed dose. Specifically, FIG. 4 compares the dissolution of the 450 mg Delafloxacin Single Layer Tablet as described in Example 3, a capsule containing 400 mg delafloxacin meglumine, the 400 mg Delafloxacin Single Layer Tablet as described in Example 2, and the 400 mg Delafloxacin Bilayer Tablet as described in Example 1.

The compositions disclosed herein exhibited a comparable dissolution profile as compared to a formulation of delafloxacin meglumine in a capsule, as shown in Table 7. Specifically, the 400 mg Delafloxacin Bilayer Tablet disclosed in Example 1 and the 400 mg Delafloxacin Single Layer Tablet disclosed in Example 2 exhibited satisfactory dissolution profile as compared to the delafloxacin API in a capsule, while the 450 mg Delafloxacin Single Layer Tablet disclosed in Example 3 exhibited an improved dissolution profile (based on amount dissolved) as compared to the delafloxacin API in a capsule. Importantly, the compositions disclosed herein dissolve much more quickly than traditional tablets, which is the first step for absorption of the API into the intestine.

**TABLE 7**

| **Dissolution Profile of Delafloxacin Tablets** | | | | |
|---|---|---|---|---|
| **Time (min)** | **API in capsule, 400 mg** | **Bilayer tablets, 400 mg** | **Single layer tablets, 400 mg** | **Single layer tablets, 450 mg** |
| | **% dissolved (% to label claim)** | | | |
| 0 | 0 | 0 | 0 | 0 |
| 7.5 | 86 | 40 | 69 | 70 |
| 15 | 87 | 56 | 75 | 77 |
| 30 | 89 | 65 | 79 | 79 |
| 45 | 89 | 68 | 82 | 81 |
| 60 | 90 | 78 | 82 | 83 |
| 75 (infinity) | 94 | 95 | 86 | 98 |

| | **Amount Dissolved, mg** | | | |
|---|---|---|---|---|
| 0 | 0 | 0 | 0 | 0 |
| 7.5 | 344 | 160 | 276 | 315 |
| 15 | 348 | 224 | 300 | 346.5 |
| 30 | 356 | 260 | 316 | 355.5 |
| 45 | 356 | 272 | 328 | 364.5 |
| 60 | 360 | 312 | 328 | 373.5 |
| 75 | 376 | 380 | 344 | 441 |
| Clinical exposure (AUC) | 24.3 | 17.6 | 18.0 | 24.0 |

### EXAMPLE 5

Compositions of the present invention were evaluated in a preclinical animal study. administered to mini-pigs in a preclinical animal study. The test formulations were formulated as dosing solutions for oral administration gavage to non-naive male Gottingen mini-pigs, approximately four months old and weighing 10-12.8 kilograms at the outset of the study. Group Assignments and dose levels are provided in Table 8.

**TABLE 8**

| **Group** | **Delafloxacin Meglumine Salt Dose** | **Delafloxacin Meglumine Salt Concentration** | **Dose #1 Volume** | **Dose #2 Volume** | **Number of Animals** |
|---|---|---|---|---|---|
| | (mg/kg/day) | (mg/mL) | (mL/kg) | (mL/kg) | Male |
| 1. Control (vehicle) | 0 | 0 | 5.0 | - | 3 |
| 2. Delafloxacin meglumine salt | 432 | 86.4 | 5.0 | - | 3 |
| 3. Delafloxacin meglumine salt | 432 | 86.4 | 5.0 | 1 tablet/day | 3 |
| + effervescence* | 150 | 30 | | | |

| | | | | | |
|---|---|---|---|---|---|
| *Group 3 animals received 1 effervescence tablet immediately after the test article formulation | | | | | |

Animals in Groups 1 and 2 were dosed once daily with vehicle control or delafloxacin meglumine salt for seven consecutive days. Animals in Group 3 were given delafloxacin meglumine salt, followed by the effervescent treatment, daily for seven consecutive days. Mortality and clinical observations were evaluated daily and for thirteen days following completion of dosing. The animals that received the combination of delafloxacin meglumine salt and effervescence *(i.e.* the effervescent agent) showed subtle improvements in the severity, incidence, onset and resolution of abnormal fecal consistency. For example, animals in Group 1 had loose feces during the dosing phase, whereas animals in Group 3 only exhibited soft feces, which is the least severe category of abnormal fecal consistency. There were individual animals in Group 3 that did not exhibit abnormal fecal consistency throughout the study. Following completion of dosing, abnormal fecal consistency resolved somewhat faster in Group 3 than in Group 2. Plasma exposure was consistent across the non-control groups.

### EXAMPLE 6

Compositions of the present invention were administered to human subjects in a clinical trial. The subjects were administered bilayer tablets, *see* Example 1, above, containing delafloxacin meglumine API (400 mg delafloxacin per capsule on a free acid basis) and the effervescent agent.

The following dosing regimen was followed in this nine (9) day clinical trial. On days 1 and 2, the subjects were administered placebo twice daily (BID). On day 3, the subjects were administered the bilayer tablet. On days 4 through 9, the subjects were administered the bilayer tablet twice daily, twelve hours apart, under fasted conditions.

Various evaluations and assessments were made during the course of the study including drug blood levels for determining pharmacokinetic/pharmacodynamic (PK/PD) parameters, incidence of diarrhea (both self-reported and clinical assessment using the standard Bristol stool chart), and adverse event reporting.

In clinical trials, administration of delafloxacin with an effervescent agent resulted in reduced side effects, as compared to administration of delafloxacin in the absence of an effervescent agent, as shown in Table 9. Specifically, Table 9 shows improved properties for a bilayer tablet of delafloxacin and an effervescent agent (*i.e.* Regimen C) as compared to compositions of delafloxacin without an effervescent agent (*i.e.* Regimen A, a capsule containing delafloxacin meglumine, and Regimen B, a capsule containing delafloxacin potassium).

The most frequently reported Treatment-Emergent Adverse Events (TEAEs) overall in this trial were classified as gastrointestinal disorders (16.7%), with diarrhea the most commonly reported TEAE (11.7%), followed by abdominal pain (5.0%). The highest percentage of subjects who reported diarrhea received oral delafloxacin meglumine salt capsules or oral delafloxacin potassium salt capsules (15.0% each), with subjects who received an oral delafloxacin meglumine salt bilayer tablet reporting fewer incidences of diarrhea (5.0%). Abdominal pain, the second most commonly reported TEAE, was reported most by subjects who received oral delafloxacin potassium salt capsules (10.0%) followed by subjects who received an oral delafloxacin meglumine salt bilayer tablet (5.0%).

**TABLE 9**

| **GI Adverse Events Reported by Two or More Subjects (Safety Population)** | | | | |
|---|---|---|---|---|
| **Oral Delafloxacin 400 mg** | | | | |
| | **Meglumine Salt (A¹)** | **Potassium Salt (B¹)** | **Meglumine Salt Bilayer Tablet (C¹)** | **Overall** |
| | **n = 20** | **n = 20** | **n = 20** | **n = 60** |
| Gastrointestinal disorders | 5 (25.0%) | 3 (15.0%) | 2 (10.0%) | 10 (16.7%) |
| Diarrhea | 3 (15.0%) | 3 (15.0%) | 1 (5.0%) | 7 (11.7%) |
| Abdominal Pain | 0 | 2 (10.0%) | 1 (5.0%) | 3 (5.0%) |
| Nausea | 0 | 1 (5.0%) | 0 | 1 (1.7%) |
| AUC (0-inf) (µg^{∗}hr/mL)² | 24.6 ± 6.5 | 11.6 ± 2.4 | 17.8 ± 5.2 | - |

| | | | | |
|---|---|---|---|---|
| ¹ Regimen A: 400-mg delafloxacin meglumine salt (4 × 100-mg capsules) Regimen B: 400-mg delafloxacin potassium salt (4 × 100-mg capsules) Regimen C: 400-mg delafloxacin meglumine salt bilayer tablet (1 × 400-mg tablet) ² Reported as a mean value. | | | | |

Blood samples for the pharmacokinetic (PK) analysis of delafloxacin were collected from each subject in all groups. Noncompartmental PK analysis was performed on the plasma concentration data using Phoenix® WinNonlin® version 6.1 (Certara, St. Louis, Missouri). AUCs were calculated as mean plus or mius standard deviation, rounded to the tenths, using the linear trapezoidal rule. TEAE are reported as frequency and percent of subjects that exhibited the TEAE.

The exposure (*i.e.* AUC) and TEAE data shown in Table 9 further demonstrate the advantages of the compositions disclosed herein. For example, while Regimen C demonstrated about 30% less exposure as compared to Regimen A, patients receiving Regimen C reported fewer GI side effects than those receiving Regimen A. Because the dosage of delafloxacin meglumine was the same in Regimens A and C, this means a larger percentage of the drug was present in the gut of patients receiving Regimen C as compared to Regimen A. A larger percentage of drug in the gut would be expected to result in a higher rate of TEAEs, but in this case the exact opposite was seen. These data evince the profound effect of the disclosed effervescent formulations at reducing GI adverse events. Additionally, Regimen B demonstrated approximately 35% less exposure than Regimen C, meaning patients receiving Regimen B had less drug in the gut as compared to Regimen C. However, Regimen B actually results in increased side effects as compared to Regimen C. Again, these date show the profound and unexpected effect of the claimed effervescent formulations. Without wishing to be bound by theory, the inventors postulate that air bubbles evolving as the compositions disclosed herein dissolve assist the drug granules in passing through the stomach into the intestine providing for a lower residence time in the patient's stomach as compared to a tablet lacking an effervescent agent. This decreased residence time in the stomach is postulated to result in the surprisingly reduced GI side effects demonstrated by the compositions disclosed herein.

The foregoing compositions are useful for oral administration to a patient for treating, preventing, or reducing the risk of a microbial infection.

## Claims

1. A pharmaceutical composition comprising:
(a) a quinolone carboxylic acid derivative or a pharmaceutically acceptable salt or ester thereof; and
(b) an effervescent agent;
wherein said quinolone carboxylic acid derivative corresponds to the following compound (A), 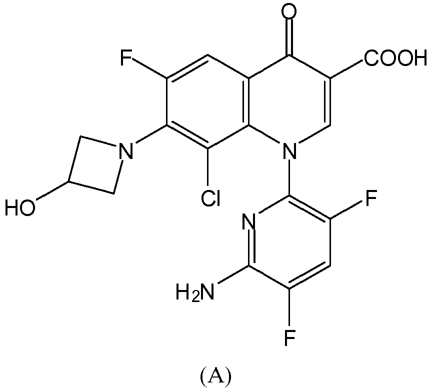 or a pharmaceutically acceptable salt or ester thereof; and
wherein said effervescent agent comprises a mixture of sodium bicarbonate, sodium dihydrogen phosphate, and citric acid.

2. The pharmaceutical composition of claim 1, wherein said quinolone carboxylic acid derivative is a D-glucitol, 1-deoxy-1-(methylamino)-, 1-(6-amino-3,5-difluoro-2-pyridinyl)-8-chloro-6-fluoro-1,4-dihydro-7-(3-hydroxy-1-azetidinyl)-4-oxo-3-quinolinecarboxylate.

3. The pharmaceutical composition of claim 2, wherein said quinolone carboxylic acid derivative is a crystalline D-glucitol, 1-deoxy-1-(methylamino)-, 1-(6-amino-3,5-difluoro-2-pyridinyl)-8-chloro-6-fluoro-1,4-dihydro-7-(3-hydroxy-1-azetidinyl)-4-oxo-3-quinolinecarboxylate **characterized by** an X-ray powder diffraction pattern substantially in accordance with that shown in FIG. 1, wherein the pattern is obtained from a copper radiation source (Cu-Kα 40 kV, 4 mA).

4. The pharmaceutical composition of claim 3, wherein said crystalline form is **characterized by** an X-ray powder diffraction pattern having peaks at 6.35, 12.70, 19.10 and 20.50 degrees 2θ, wherein the pattern is obtained from a copper radiation source (Cu-Kα, 40 kV, 4 mA); a melting point of 168-171 °C; or the differential scanning calorimetry thermogram shown in FIG. 3.

5. The pharmaceutical composition of any one of claims 1 to 2 further comprising a polyhydroxyamine compound.

6. The pharmaceutical composition of claim 5, wherein said polyhydroxyamine compound is meglumine.

7. A pharmaceutical composition of claim 1 comprising:
(a) from 100 mg to 750 mg of said quinolone carboxylic acid derivative, on an acid active basis, wherein said quinolone carboxylic acid derivative is delafloxacin or a pharmaceutically acceptable salt thereof; and
(b) from 100 mg to 500 mg of said effervescent agent.

8. A pharmaceutical composition of claim 1 comprising:
(a) 650 mg of said quinolone carboxylic acid derivative, wherein said quinolone carboxylic acid is delafloxacin meglumine; and
(b) 150 mg of said effervescent agent.

9. The pharmaceutical composition of any one of claims 1 to 8 which is a unit dosage.

10. The pharmaceutical composition of any one of claims 1 to 8 in the form of a tablet.

11. The pharmaceutical composition of claim 10, wherein the tablet is a single layer tablet.

12. The pharmaceutical composition of claim 10, wherein the tablet is a bilayer tablet comprising a first layer and a second layer.

13. The pharmaceutical composition of claim 12, wherein a first layer comprises said quinolone carboxylic acid derivative or a pharmaceutically acceptable salt thereof and said second layer comprises said effervescent agent.

14. The pharmaceutical composition of any one of claims 1 to 8 in the form of a capsule.

15. The pharmaceutical composition of any one of claims 1 to 8, wherein said quinolone carboxylic acid derivative and said effervescent agent are administered as separate dosage forms.

## Patentansprüche

1. Pharmazeutische Zusammensetzung, umfassend:
(a) ein Chinoloncarbonsäurederivat oder ein pharmazeutisch unbedenkliches Salz oder ein pharmazeutisch unbedenklicher Ester davon und
(b) ein Brausemittel,
wobei das Chinoloncarbonsäurederivat der folgenden Verbindung (A) 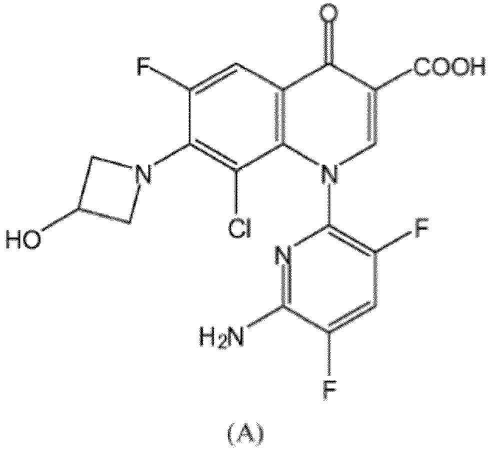 oder einem pharmazeutisch unbedenklichen Salz oder pharmazeutisch unbedenklichen Ester davon entspricht und wobei das Brausemittel eine Mischung aus Natriumhydrogencarbonat, Natriumdihydrogenphosphat und Citronensäure umfasst.

2. Pharmazeutische Zusammensetzung nach Anspruch 1, wobei es sich bei dem Chinoloncarbonsäurederivat um ein D-Glucitol-1-desoxy-1-(methylamino)-1-(6-amino-3,5-difluor-2-pyridinyl)-8-chlor-6-fluor-1,4-dihydro-7-(3-hydroxy-1-azetidinyl)-4-oxo-3-chinolincarboxylat handelt.

3. Pharmazeutische Zusammensetzung nach Anspruch 2, wobei es sich bei dem Chinolonsäurederivat um ein kristallines D-Glucitol-1-desoxy-1-(methylamino)-1-(6-amino-3,5-difluor-2-pyridinyl)-8-chlor-6-fluor-1,4-dihydro-7-(3-hydroxy-1-azetidinyl)-4-oxo-3-chinolincarboxylat handelt, das durch ein Röntgenpulverbeugungsspektrum im Wesentlichen gemäß demjenigen gekennzeichnet ist, das in FIG. 1 gezeigt ist, wobei das Spektrum von einer Kupferstrahlungsquelle (Cu-Kα, 40 kV, 4 mA) erhalten wird.

4. Pharmazeutische Zusammensetzung nach Anspruch 3, wobei die kristalline Form durch ein Röntgenpulverbeugungsspektrum mit Peaks bei 6,35, 12,70, 19,10 und 20,50 Grad 2θ, wobei das Spektrum von einer Kupferstrahlungsquelle (Cu-Kα, 40 kV, 4 mA) erhalten wird, einen Schmelzpunkt von 168-171°C oder das in FIG. 3 gezeigte Differentialscanningkalorimetrie-Thermogramm gekennzeichnet ist.

5. Pharmazeutische Zusammensetzung nach einem der Ansprüche 1 bis 2, weiterhin umfassend eine Polyhydroxyaminverbindung.

6. Pharmazeutische Zusammensetzung nach Anspruch 5, wobei es sich bei der Polyhydroxyaminverbindung um Meglumin handelt.

7. Pharmazeutische Zusammensetzung nach Anspruch 1, umfassend:
(a) 100 mg bis 750 mg des Chinoloncarbonsäurederivats, bezogen auf aktive Säure, wobei es sich bei dem Chinoloncarbonsäurederivat um Delafloxacin oder ein pharmazeutisch unbedenkliches Salz davon handelt, und
(b) 100 mg bis 500 mg des Brausemittels.

8. Pharmazeutische Zusammensetzung nach Anspruch 1, umfassend:
(a) 650 mg des Chinoloncarbonsäurederivats, wobei es sich bei dem Chinoloncarbonsäurederivat um Delafloxacin-Meglumin handelt, und
(b) 150 mg des Brausemittels.

9. Pharmazeutische Zusammensetzung nach einem der Ansprüche 1 bis 8, bei der es sich um eine Einheitsdosis handelt.

10. Pharmazeutische Zusammensetzung nach einem der Ansprüche 1 bis 8 in Form einer Tablette.

11. Pharmazeutische Zusammensetzung nach Anspruch 10, wobei es sich bei der Tablette um eine Einschichttablette handelt.

12. Pharmazeutische Zusammensetzung nach Anspruch 10, wobei es sich bei der Tablette um eine eine erste Schicht und eine zweiten Schicht umfassende Zweischichttablette handelt.

13. Pharmazeutische Zusammensetzung nach Anspruch 12, wobei die erste Schicht das Chinoloncarbonsäurederivat oder ein pharmazeutisch unbedenkliches Salz davon umfasst und die zweite Schicht das Brausemittel umfasst.

14. Pharmazeutische Zusammensetzung nach einem der Ansprüche 1 bis 8 in Form einer Kapsel.

15. Pharmazeutische Zusammensetzung nach einem der Ansprüche 1 bis 8, wobei das Chinoloncarbonsäurederivat und das Brausemittel als getrennte Dosierungsformen verabreicht werden.

## Revendications

1. Composition pharmaceutique comprenant :
(a) un dérivé de quinolone de type acide carboxylique ou un sel pharmaceutiquement acceptable ou ester de celui-ci ; et
(b) un agent effervescent ;
dans laquelle ledit dérivé de quinolone de type acide carboxylique correspond au composé (A) suivant, 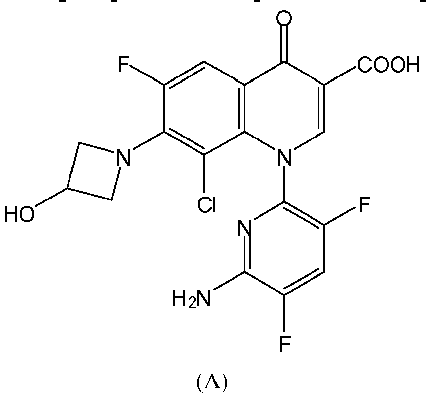 ou à un sel ou ester pharmaceutiquement acceptable de celui-ci ; et
dans laquelle ledit agent effervescent comprend un mélange de bicarbonate de sodium, de dihydrogénophosphate de sodium et d'acide citrique.

2. Composition pharmaceutique selon la revendication 1, dans laquelle ledit dérivé de quinolone de type acide carboxylique est un D-glucitol, 1-désoxy-1-(méthylamino)-, 1-(6-amino-3,5-difluoro-2-pyridinyl)-8-chloro-6-fluoro-1,4-dihydro-7-(3-hydroxy-1-azétidinyl)-4-oxo-3-quinoléinecarboxylate.

3. Composition pharmaceutique selon la revendication 2, dans laquelle ledit dérivé de quinolone de type acide carboxylique est un D-glucitol, 1-désoxy-1-(méthylamino)-, 1-(6-amino-3,5-difluoro-2-pyridinyl)-8-chloro-6-fluoro-1,4-dihydro-7-(3-hydroxy-1-azétidinyl)-4-oxo-3-quinoléinecarboxylate cristallin **caractérisé par** un diagramme de diffraction X de poudre substantiellement en accord avec celui présenté sur la FIG. 1, dans laquelle le diagramme est obtenu à partir d'une source de rayonnement à base de cuivre (Cu-Kα 40 kV, 4 mA).

4. Composition pharmaceutique selon la revendication 3, dans laquelle ladite forme cristalline est **caractérisée par** un diagramme de diffraction X de poudre présentant des pics à 6,35, 12,70, 19,10 et 20,50 degrés 2θ, dans laquelle le diagramme est obtenu à partir d'une source de rayonnement à base de cuivre (Cu-Kα, 40 kV, 4 mA) ; un point de fusion de 168 à 171 °C; ou le thermogramme de calorimétrie différentielle à balayage présenté sur la FIG. 3.

5. Composition pharmaceutique selon l'une quelconque des revendications 1 à 2 comprenant en outre une polyhydroxyamine.

6. Composition pharmaceutique selon la revendication 5, dans laquelle ladite polyhydroxyamine est la méglumine.

7. Composition pharmaceutique selon la revendication 1 comprenant :
(a) entre 100 mg et 750 mg dudit dérivé de quinolone de type acide carboxylique, en se basant sur l'acide actif, dans laquelle ledit dérivé de quinolone de type acide carboxylique est la délafloxacine ou un sel pharmaceutiquement acceptable de celui-ci ; et
(b) entre 100 mg et 500 mg dudit agent effervescent.

8. Composition pharmaceutique selon la revendication 1 comprenant :
(a) 650 mg dudit dérivé de quinolone de type acide carboxylique, dans laquelle ladite quinolone de type acide carboxylique est la délafloxacine méglumine ; et
(b) 150 mg dudit agent effervescent.

9. Composition pharmaceutique selon l'une quelconque des revendications 1 à 8 qui est une dose unitaire.

10. Composition pharmaceutique selon l'une quelconque des revendications 1 à 8 sous la forme d'un comprimé.

11. Composition pharmaceutique selon la revendication 10, dans laquelle le comprimé est un comprimé monocouche.

12. Composition pharmaceutique selon la revendication 10, dans laquelle le comprimé est un comprimé bicouche comprenant une première couche et une seconde couche.

13. Composition pharmaceutique selon la revendication 12, dans laquelle une première couche comprend ledit dérivé de quinolone de type acide carboxylique ou un sel pharmaceutiquement acceptable de celui-ci et ladite seconde couche comprend ledit agent effervescent.

14. Composition pharmaceutique selon l'une quelconque des revendications 1 à 8 sous la forme d'une capsule.

15. Composition pharmaceutique selon l'une quelconque des revendications 1 à 8, dans laquelle ledit dérivé de quinolone de type acide carboxylique et ledit agent effervescent sont administrés sous des formes galéniques distinctes.
